# EUROPEAN PATENT SPECIFICATION

(11) **EP 2 291 545 B1**
(45) Date of publication and mention of the grant of the patent: **08.05.2013**
(21) Application number: 09762774.9
(22) Date of filing: 12.06.2009
(51) Int. Cl.: C12Q 1/68

(54) **INFLUENZA SEQUENCES**
INFLUENZA-SEQUENZEN
SÉQUENCES DE LA GRIPPE

(30) Priority: 14.06.2008 SG 200804538
(43) Date of publication of application: 09.03.2011
(73) Proprietor: Veredus Laboratories Pte Ltd, Singapore 118258 (SG)
(72) Inventor: ONG, Kian, Leong, Singapore 118258 (SG); TAN, Sok, Pin, Singapore 118258 (SG)
(74) Representative: Icely, Dominic Michael
(86) International application number: PCT/SG2009/000212
(87) International publication number: WO 2009/151407

(56) References cited:
- WO-A1-00/17391
- WO-A1-02/19118
- WO-A2-2007/058629
- WO-A2-2007/130519
- WO-A2-2008/042450
- WO-A2-2008/054830
- US-A1- 2007 224 594
- US-A1- 2008 124 710
- WU ET AL: "A multiplex real-time RT-PCR for detection and identification of influenza virus types A and B and subtypes H5 and N1", 19 December 2007 (2007-12-19), JOURNAL OF VIROLOGICAL METHODS, ELSEVIER BV, NL, PAGE(S) 81 - 88, XP022487596, ISSN: 0166-0934 * page 85, column 1, paragraph 1 * * the whole document *
- LODES, M. J ET AL.: 'Use of Semiconductor-Based Oligonucleotide Microarrays for Influenza A Virus Subtype Identification and Sequencing' JOURNAL OF CLINICAL MICROBIOLOGY vol. 44, no. 4, 2006, pages 1209 - 1218, XP008139228
- QUAN P-L. ET AL: 'Detection of Respiratory Viruses and Subtype Identification of Influenza A Viruses by GreeneChipResp Oligonucleotide Microarray' JOURNAL OF CLINICAL MICROBIOLOGY vol. 45, no. 8, 2007, pages 2359 - 2364, XP008139242
- GYARMATI P. ET AL: 'Simultaneous Genotyping of All Hemagglutinin and Neuraminidase Subtypes of Avian Influenza Viruses by Use of Padlock Probes' JOURNAL OF CLINICAL MICROBIOLOGY vol. 46, no. 5, 2008, pages 1747 - 1751, XP008139239

## Description

### FIELD

The present invention relates to the fields of medicine, cell biology, molecular biology and genetics. More particular, the invention relates to methods and nucleic acid sequences suitable for use in detecting a pathogen or products thereof in a sample.

### BACKGROUND

Influenza is a frequently underestimated infectious disease which can result in high morbidity and mortality rates especially in elderly persons and in high-risk patients. Influenza A and influenza B viruses are responsible for genuine virus influenza which is contracted by several 100 million persons worldwide each year. The influenza A and B viruses primarily infect the nasopharyngeal and oropharyngeal cavities and initially cause general respiratory symptoms in the affected persons. It is not possible even for experienced medical professionals to very reliably diagnose influenza solely on the basis of the patient's clinical symptoms since other viruses which infect the nasal or pharyngeal cavity such as adenoviruses, parainfluenza viruses or respiratory syncitial viruses (RS viruses) cause similar symptoms.

Influenza A is an infectious disease of animals caused by type A strains of the influenza virus that normally infect birds, and less commonly, pigs. There are many subtypes of the influenza A virus. These subtypes are based on the haemagglutinin (HA) segment, which has 16 varieties (H1-H16) and neuraminidase (NA) segment, which has 9 varieties (N1-N9). The reason for the high degree of genetic and hence immunological variability especially of the influenza A viruses is due to the fact that a genetic shift (reassortment of the viral genes) can also occur in rare cases in addition to the usual genetic drift (point mutation). This is due to the fact that, in contrast to other viruses, the genome of the influenza viruses is segmented and that influenza A is a pathogen in humans as well as in animals. Influenza B infects only humans.

Due to the high medical importance of influenza infections, almost every country in the world now has a nationally organized influenza monitoring system. For this scheme general practitioners remove swabs from the nose and/or throat and send them to the respective national reference centre. The influenza A/B viruses are then usually detected by eluting the swabs and subsequently culturing the patient specimens on mammalian cells such as MDCK cells (Madine-Darby Canine Kidney cells). The culture in these special laboratories can take up to 14 days and is thus not of immediate relevance for the diagnosis of the individual patients. Rather the goal of the national reference centres is to type and subtype the cultured viruses and to report the results to the World Health Organisation (WHO). The job of the vaccine manufacturer is then to adapt next year's influenza vaccines to the latest circulating viral strains on the basis of the annual WHO recommendation.

Rapid and reliable detection of influenza A virus infection is therefore very important. Current laboratory methods of detecting influenza A and B infections commonly involve antigen detection, isolation in cell culture, or detection of influenza-specific RNA by reverse transcriptase polymerase chain reaction (RT-PCR). There is a huge problem in using antigen detection due to the sensitivity of such tests. The rapid test kits generally provide results within 24 hours and are approximately 70% sensitive for detecting influenza and approximately 90% specific. The sensitivity of the rapid test kits means that as many as 30% of samples may yield false negatives, and the tests are not multiplexed. Each of these assay techniques has advantages and disadvantages that make them more or less suitable for use in public health laboratories, or hospital-based laboratories, but none of these existing assays are currently employed at point-of care: They are all conducted in a laboratory and usually results are not produced rapidly enough to impact on the prescribed treatment. Virus isolation in cell culture is both laborious and time intensive.

Currently as stipulated by the World Health Organization, recommended diagnostic methods for the identification of avian influenza A virus in human are immunofluorescence assay (IFA), virus culture and PCR assays. Both IFA and virus culture methods are laborious and are not feasible for large amounts of samples.

WO 00/17391 describes a multiplex RT-PCR method and primers for determining the presence of microorganisms which infect the respiratory tract. WO 2008/042450 describes the multiplex detection of respiratory pathogens in a sample. US 2007/0224594 describes methods for detecting influenza virus. Wu et al. (Journal of Virological Methods, 148 (2008) 81-88) describes a novel RT-PCR method for detecting influenza A subtypes. WO 2008/054830 describes an array having capture probes for detecting and diagnosing the presence of influenza virus.

The threat of an influenza pandemic has propelled the need of a new influenza diagnostic test system that should be able to do fast and reliable detection of human influenza, with the capability to identify and differentiate seasonal influenza A and B from novel influenza A as well as detecting newly emergent influenza A strains.

Rapid specific and sensitive detection and typing of influenza virus and identification of its various strains is critical to identification and control of a potential human pandemic. There is also a perceived need for an informative influenza assay that can be performed in the field, i.e., at the point of care ("POC").

### SUMMARY

According to a 1^{st} aspect of the present invention, we provide a combination of nucleic acids comprising primers of SEQ ID NO: 1 to SEQ ID NO: 16 as shown in Table D1, or a variant, homologue, derivative or fragment of at least 20 nucleotides in length thereof having at least 90% sequence identity thereto, for nucleic acid amplification of an influenza virus sequence, and probes of SEQ ID NO: 17 to SEQ ID NO: 53 as shown in Table D2, or a variant, homologue, derivative or fragment of at least 20 nucleotides in length thereof having at least 90% sequence identity thereto, wherein the probes are immobilised and are capable of hybridising to an influenza virus sequence.

The combination may further comprise one or more of the primer sequences set out in Table D3.

The combination may further comprise one or more of the probe sequences set out in Table D4.

The probes may be immobilised in the form of an array such as a microarray.

The combination may be used on a device comprising a first chamber for nucleic acid amplification of the influenza virus sequence using the primers and a second chamber for nucleic acid hybridisation of the influenza virus sequence using the immobilised probes. The first and second chambers of the device may be in fluid connection.

According to a 2^{nd} aspect of the present invention, there is provided a method of detecting an influenza virus sequence in a biological sample and/or determining the strain of an influenza virus sequence in a biological sample, comprising:
a) performing a nucleic acid amplification comprising amplifying the influenza virus sequence to produce amplicons using primers of SEQ ID NO: 1 to SEQ ID NO: 16 as shown in Table D1, or a variant, homologue, derivative or fragment of at least 20 nucleotides in length thereof having at least 90% sequence identity thereto;
b) performing a nucleic acid hybridisation comprising hybridising the amplified influenza virus sequence to immobilised probes of SEQ ID NO: 17 to SEQ ID NO: 53 as shown in Table D2, or a variant, homologue, derivative or fragment of at least 20 nucleotides in length thereof having at least 90% sequence identity thereto; and
c) detecting binding between the immobilised probe and the amplified influenza virus sequence.

The nucleic acid amplification step a) may be performed in a first chamber of a device, and the nucleic acid hybridisation step b) may be performed in a second chamber of the device.

The method may be capable of multiplex detection of targets.

The nucleic acid amplification step a) may further comprise amplifying the influenza sequence using one or more of the primer sequences set out in Table D3.

The nucleic acid hybridisation step b) may further comprise hybridising the influenza sequence using one or more of the probe sequences set out in Table D4.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies : A Laboratory Manual: Portable Protocol NO. I by Edward Harlow, David Lane, Ed Harlow (1999, Cold Spring Harbor Laboratory Press, ISBN 0-87969-544-7); Antibodies : A Laboratory Manual by Ed Harlow (Editor), David Lane (Editor) (1988, Cold Spring Harbor Laboratory Press, ISBN 0-87969-314-2), 1855. Handbook of Drug Screening, edited by Ramakrishna Seethala, Prabhavathi B. Fernandes (2001, New York, NY, Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3.

### BRIEF DESCRIPTION OF THE FIGURES

Figure 1 is a fluidic diagram that illustrating a Lab-on-Chip described here. A. VereChip- Lab-on-Chip B. VereID BioSystem.
Figure 2 is a diagram showing an overview of VereFlu^{™} Influenza A/B Detection.
Figure 3 is a diagram showing the VereFlu Microarray Probe Panel.
Figure 4 is a diagram showing the specificity of the probes on the microarray panel for the HA gene of the H5 type/ H5N1 subtype of influenza A.
Figure 5 is a diagram showing the specificity of the probes on the microarray panel for the NA gene of the H5 type/ H5N1 subtype of influenza A.
Figure 6 is a diagram showing the specificity of the probes on the microarray panel for H7 type of influenza A.
Figure 7 is a diagram showing the specificity of the probes on the microarray panel for H9 type/ H9N2 subtype of influenza A.
Figure 8 is a representative microarray image for H5N 1 influenza virus detected by the VereFlu microarray.
Figure 9A is a diagram showing tip placement into an inlet hole during sample loading. Figure 9B is a diagram showing a pipette sketch indicating the different stop positions.
Figure 10 is a diagram showing IN and PCR sealing clamps.
Figure 11 is a diagram showing a bottom view of IN (left) and PCR (right) clamps.
Figure 12 is a diagram showing alignment holes for IN and PCR (or Hyb) clamps.
Figure 13 is a diagram showing a closed chip ready for the PCR thermal cycles.
Figure 14 is a diagram showing microarray chamber filling. Left: Microarray chamber is half filled after loading only one inlet. Right: Microarray chamber completely filled.
Figure 15 is a diagram showing a clamp for the detection area sealing during the hybridization phase (bottom view).
Figure 16A is a diagram showing a first sealing step during hybridization phase, placing inlet clamp. Figure 16B is a diagram showing a closed chip ready for the hybridization.

### DETAILED DESCRIPTION

As noted in the "Background" section, the increasingly viable threat of an epidemic and/or pandemic drives the need for a fast and reliable detection of human influenza, with the capability to detect, identify and differentiate seasonal influenza A and B from novel influenza A as well as detecting newly emergent influenza A strains.

We describe a nucleic acid sequence as shown in Table D1 or Table D2, or a variant, homologue, derivative or fragment thereof. The sequence may comprise a sequence set out in Table D3 or Table D4. A combination of two or more sequences is also described. The nucleic acid sequence or combination may be used for detection of influenza or discrimination between strains of influenza.

The methods and compositions described here enable influenza assays to be performed in the field, i.e., at the point of care. POC assays compatible with more extensive laboratory analysis, such as sequencing of, for example, HA and NA may enable savings in time and money. This may also allow the evolution of a viral disease and viral genomics to be analyzed in real-time.

The methods and compositions described here may provide a rapid, sensitive and accurate method to detect, differentiate and identify various sub-types and strains of an influenza A and/or B virus. The methods and compositions described here may provide signature sequences of various influenza A and B viruses that may be useful in detecting the presence and identification of a particular influenza strain and/or sub-type. The nucleic acid sequences described here may be used to detect for example influenza A, influenza B, and the like.

The methods and compositions described here also enable use of polymerase chain reaction for the detection of influenza infection. PCR is widely recommended and used due to its high sensitivity and specificity. PCR tests are used as both testing and confirmation in the detection of H5N1. RT-PCR is increasingly becoming popular due to its specificity, sensitivity and turn-around time. By using RT-PCR techniques with defined primers, it is possible to provide a viral detection as well as subtype identification.

The methods and compositions described here may allow fast and reliable detection of human influenza, with the capability to identify and differentiate seasonal influenza A and B from novel influenza A as well as detecting newly emergent influenza A strains.

We describe as an example a test system comprising a plurality of PCR primer pairs, used to reverse transcribe low amounts of influenza viral RNA from a sample, and amplify them, and a plurality of probes to hybridize amplicons for the accurate detection, differentiation and identification of the influenza strains and subtypes.

### INFLUENZA SEQUENCES

We describe a number of nucleic acid sequences. The sequences may be used for any means for which nucleic acid sequences are suitable. For simplicity, the nucleic acid sequences the subject of this disclosure may be referred to as "influenza sequences".

The "influenza sequence" may comprise a sequence corresponding to a portion of an influenza nucleic acid sequence, for example, an influenza gene. The influenza gene may comprise a gene from or of an influenza virus. The "influenza sequences" may further comprise other nucleic acid residues which have no such correspondence.

The influenza sequence may comprise a sequence set out in Table D1 (SEQ ID NO: 1 to SEQ ID NO: 16) or a variant, homologue, derivative or fragment of at least 20 nucleotides in length thereof having at least 90% sequence identity thereto. The influenza sequence may also comprise a sequence set out in Table D2 (SEQ ID NO: 17 to SEQ ID NO: 53) or a variant, homologue, derivative or fragment of at least 20 nucleotides in length thereof having at least 90% sequence identity thereto.

The influenza sequence may comprise a sequence set out in Table D3. The influenza sequence may comprise a sequence set out in Table D4. The influenza sequence may comprise a variant, homologue, derivative or fragment of any such sequence.

Combinations of any of these sequences are also described. We describe pairwise combinations of the sequences in Table D1, for example, a combination of SEQ ID NO: 1 and SEQ ID NO: 2, a combination of SEQ ID NO: 3 and SEQ ID NO: 4, a combination of SEQ ID NO: 5 and SEQ ID NO: 6, a combination of SEQ ID NO: 7 and SEQ ID NO: 8, a combination of SEQ ID NO: 9 and SEQ ID NO: 10, a combination of SEQ ID NO: 11 and SEQ ID NO: 12, a combination of SEQ ID NO: 13 and SEQ ID NO: 14, and a combination of SEQ ID NO: 15 and SEQ ID NO: 16. Combinations of such combinations are also described.

We also describe a combination comprising SEQ ID NO: 1 to SEQ ID NO: 14, and optionally SEQ ID NO: 15 and SEQ ID NO: 16, as shown in Table D 1. We further describe a combination comprising all the sequences shown in Table D1, i.e., SEQ ID NO: 1 to SEQ ID NO: 16.

We further describe combinations of the sequences in Table D2, for example, a combination comprising SEQ ID NO: 17 to SEQ ID NO: 53, as shown in Table D2. We further describe for a combination comprising all the sequences shown in Table D2, i.e., SEQ ID NO: 17 to SEQ ID NO: 53.

The invention relates to a combination comprising primers of SEQ ID NO: 1 to SEQ ID NO: 16 as shown in Table D1, or a variant, homologue, derivative or fragment of at least 20 nucleotides in length thereof having at least 90% sequence identity thereto, for nucleic acid amplification of an influenza virus sequence, and probes of SEQ ID NO: 17 to SEQ ID NO: 53 as shown in Table D2 or a variant, homologue, derivative or fragment of at least 20 nucleotides in length thereof having at least 90% sequence identity thereto, wherein the probes are immobilised and are capable of hybridising to an influenza virus sequence.

The influenza virus may comprise influenza A virus. The influenza virus may comprise a human influenza virus or an animal influenza virus. The influenza virus may comprise avian influenza virus ("bird flu").

The influenza gene may include the HA (hemagglutinin) gene or the NA (neuraminidase) gene.

The influenza sequences described here may be single stranded or double stranded.

The influenza sequences described here may in particular be capable of binding to a relevant influenza gene. The influenza gene may comprise DNA, RNA, mRNA, cDNA, etc, or a portion thereof. The influenza gene may include the HA (hemagglutinin) gene or the NA (neuraminidase) gene, or both. Other genes may be detected as part of the process.

The sequences may be used to detect the presence of influenza nucleic acids in a sample. They may be used to detect the presence of influenza viruses or products thereof in a sample. They may be used to detect the presence of influenza viruses of particular strains in a sample. They may be used to discriminate between these strains.

### Size of Nucleic Acids

The size of the influenza nucleic acid sequences, including primers and probes, may vary, as will be appreciated by those in the art, in general varying from 5 to 100 nucleotides in length, for example between 10 and 100, such as 15 and 50 and from 10 to 35, depending on the use and amplification technique. According to the invention, the influenza nucleic acid sequences are at least 20 nucleotides in length.

Generally the sequence, for example the probe or primer, may include a target pathogen specific sequence sufficient to confer specific amplification or hybridization to the respective influenza DNA. The nucleic acid sequences disclosed here may further comprise other components, such as adapter sequences. Where present, the adapter sequence may range for example from 15-25 nucleotides in length, for example 20. The target specific portion of the sequence may be from 15- 50 nucleotides in length. In addition, the sequence may include one or more additional amplification priming sites.

### Probe Design

For probe design, sequences of different strains of each subtype may be extracted from established databases such as NCBI and assessed for potential probe sites with the following parameters;
i. Unique sequences that can differentiate between different strains of influenza
ii. Pre-determined microarray hybridization conditions
iii. Probe melting temperature
iv. Hairpin forming potential
v. Length of probe
vi Binding Free Energy

A good probe should have maximum hybridization with its target, minimum cross-hybridization with its non-targets and a good fluorescence with for example Signal median /Background median > 3 * max (Signal med / Background med) of Negative Controls.

Probe length of 25-mer may be used to achieve a balance between specificity and tolerance for mismatches between different strains of the same subtype. The potential probes may be spotted onto the microarray and tested again. Probes that do not work or displayed cross-hybridization may be removed and redesigned. Whenever feasible, probes that span the beginning, middle and end of the target PCR fragments are selected. This is to ensure that even if mutation arises on one region of the target fragment, we can still detect the virus from the remaining two regions.

In instances where probes generated by the software do not work, probes may be designed manually. The manually designed probes may be subjected to and assessed with the same parameters by the software. However, certain compromises may have to be made to the parameters in special cases. The probes may be then spotted and analyzed again. The process may be repeated until the required number of probes and their required specifications are met.

The sequences of the different strains of each subtype may be aligned and potential probes sites for each subtype may be identified. Sites flanking regions of about 150-300 bp and containing the most number of potential probe sites may be used to design the primers. Once primer sets of each subtype are designed, they may be tested against all the other subtype controls for cross-primings in a multiplexing condition. Any primers exhibiting cross-priming tendencies may be redesigned and may be repeated until the cross-priming problem is solved. A same parallel set of experiments may be carried out on a thermal cycler and results visualized on suitable equipment such as an Agilent Bioanalizer to verify the LOC results.

### Immobilisation

The influenza sequences may be immobilised, for example by being bound to a substrate. In one embodiment of the methods and compositions described here, detection of the amplified products described above may make use of arrays.

By "substrate" or "solid support" or other grammatical equivalents we mean any material to which a capture probe can be immobilized. As will be appreciated by those in the art, the number of possible substrates is very large. Possible substrates include, but are not limited to, glass and modified or functionalized glass, plastics (including acrylics, polystyrene and copolymers of styrene and other materials, polypropylene, polyethylene, polybutylene, polyurethanes, TeflonJ, etc.), polysaccharides, nylon or nitrocellulose, resins, silica or silica-based materials including silicon and modified silicon, carbon, metals, inorganic glasses, plastics, and a variety of other polymers. In general, the substrates allow optical detection and do not themselves appreciably fluoresce.

Generally the substrate is flat, although as will be appreciated by those in the art, other configurations of substrates may be used as well.

The substrate may comprise a silicon base. The influenza sequences may be provided in a spaced conformation, for example a regular space conformation, for example in the form of an array. The array may comprise a microarray. Methods of producing nucleic acid arrays are known in the art.

The array may comprise the influenza sequences set out in Table D2. The array may comprise one or more control sequences.

### Sample

The methods and compositions described here may be used for detecting influenza viruses or sequences in a sample. The sample may comprise a biological sample. The sample may be taken from an individual, which may be a human or animal.

The sample may comprise any number of things, including, but not limited to, bodily fluids (including, but not limited to, blood, nasopharyngeal secretions, urine, serum, lymph, saliva, anal and vaginal secretions, perspiration and semen, of virtually any organism, for example mammalian samples, environmental samples (including, but not limited to, air, agricultural, water and soil samples); biological warfare agent samples; research samples; purified samples, such as purified genomic DNA, RNA, proteins, etc.; raw samples (bacteria, virus, genomic DNA, etc. As will be appreciated by those in the art, virtually any, or no, experimental manipulation may have been done on the sample.

The nucleic acid sequences described here may for example be used in a molecular diagnostic test for influenza. Such a test may be conducted for example using standard RNA extraction protocols, Reverse Transcription-Polymerase Chain Reaction (RT-PCR), etc.

### Nucleic Acid Amplification

As is outlined more fully below, probes may be made to hybridize to target sequences such as amplicons to determine the presence or absence of the target sequence in a sample. Primers may also be made to amplify target sequences to determine the presence or absence of the target sequence in a sample.

The influenza sequences may therefore be used as nucleic acid primers. The primers may comprise the influenza sequences set out in Table D1. They may further comprise one or more influenza sequences set out in Table D3.

A target sequence from a sample may be amplified to produce a secondary target, e.g. the amplicon, that may be detected by the methods described in this document. The target sequence may be any length, with the understanding that longer sequences are more specific. The complementary target sequence may also take a number of forms. For example, it may be contained within a larger nucleic acid sequence, e.g. all or part of a gene or mRNA, a restriction fragment of a plasmid or genomic DNA, among others.

The amplification reaction may comprise binding between the influenza sequence and a target. A primer nucleic acid may be contacted to the target sequence to form a hybridization complex. The primer may then be used to amplify the target sequence, as described in further detail below. By "primer nucleic acid" herein is meant a probe nucleic acid that will hybridize to some portion, i.e. a domain, of the target sequence.

The primers may be used for amplification of nucleic acids by any means known in the art. For example, PCR (polymerase chain reaction) may be employed. As another example, RT-PCR (reverse transcription - polymerase chain reaction) may be employed. Other amplification methods may also be used, for example, LCR (ligase chain reaction), dePCR (digital polymerase chain reaction), Long PCR, Quantitative End-Point PCR, Quantitative Real-Time PCR, Rapid Amplification of cDNA Ends (RACE), TMA, NASBA, etc. These methods are known in the art, and methods for employing the influenza sequences for use in such methods will be evident to the skilled reader.

The influenza sequences of the invention are provided as combinations. For example, we describe a combination of any two or more sequences set out in Table D1. The combination may comprise two sequences, corresponding to a forward primer and a reverse primer, as set out in Table D1. The combination of two sequences may include a forward primer for a particular gene and a reverse primer for that gene. Combinations of such pairwise combinations are also provided.

### Nucleic Acid Hybridisation and Detection

The amplified amplicons may be detected using probes comprising the nucleic acid sequences described here. Thus, the influenza sequences may be used as probes. The probes may comprise the influenza sequences set out in Table D2. They may further comprise one or more influenza sequences set out in Table D4.

For this purpose, the nucleic acid sequences described here, including the probes, may be designed to be complementary to a target sequence. This may comprise either the target sequence of the sample or to other probe sequences, as is described below, such that hybridization of the target sequence and the probes of the may occur. As outlined below, this complementarity need not be perfect; there may be any number of base pair mismatches which will interfere with hybridization between the target sequence and the single stranded nucleic acids as described here. However, if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under normal reaction conditions.

The influenza nucleic acid sequences described in this document may be used in solution for conventional nucleic acid hybridisation. The hybridisation may be performed under stringent hybridisation conditions or non-stringent hybridisation conditions. An example of a stringent hybridisation condition is 65°C and 0.1×SSC (1×SSC = 0.15 M NaCl, 0.015 M Na3 Citrate pH 7.0). A further example of a hybridisation condition is 48 mM phosphate Buffer, 1742.4 mM NaCl, 0.24% Tween 20, 4.8X Denhardt's solution, 2.4 nM probe or probes (e.g., 3 probes) - phosphate buffer comprises 12.96 mM KCl and 657.6 mM NaCl.

A variety of hybridization conditions may be used in the methods and compositions described here, including high, moderate and low stringency conditions. Stringent conditions are sequence-dependent and will be different in different circumstances. Longer sequences hybridize specifically at higher temperatures. The hybridization may be done at a predetermined temperature for 30 min using Phosphate Buffered Solution, NaCl, KCI, Tween 20 and Denhardt's solution.

Methods of using such nucleic acid sequence probes for the detection of target nucleic acid sequences are known in the art, and are described in, for example, Maniatis *et al.*

The probes may be labelled for the purpose of amplification or detection or both. The labelling may comprise any signal producing entity.

A label may for example comprise a chemical, moiety or molecule that allows detection of the label together with any molecule, surface or material to which the label is applied, attached, coupled, hybridized or bound to. Examples of labels include dyes, radiolabels, fluorescent labels, magnetic labels and enzymatic labels.

The label may in particular comprise a radioactive label, such as ³²P. The label may comprise a non-radioactive label such as digoxigenin. The signal may be detected by any suitable means known in the art.

The hybridisation may be performed with the influenza sequences in solution or immobilised, for example, where an array of probe sequences is employed..

### Two Chamber Chip

In some embodiments of the methods and compositions described here, a two component or two chamber system may be used. For example, the methods described here may be carried out on a "chip", referred to for convenience as a VereChip.

In a two chamber system, a first chamber or compartment may comprise a space for nucleic acid amplification. The first chamber may therefore comprise a micro-reactor, such as a nucleic acid amplification micro-reactor chamber, e.g., a PCR micro-reactor. The first chamber may be used for reverse transcription (see below).

A second chamber may comprise a space for nucleic acid hybridisation. The second chamber may therefore comprise a hybridization chamber. The second chamber may comprise immobilised probes for example in the form of an array.

The two chambers may be connected, for example fluidly connected such that fluid may flow from one chamber to the other. The flow may be controlled. The flow may be one way; for example, fluid may be allowed to flow from the first chamber to the second but prevented from flowing from the second chamber to the first. The chip may comprise a valve or stopper for this purpose.

Where a method using such a two chamber chip is employed, nucleic acid amplification may be conducted in the first chamber to produce amplicons. The amplicons are then moved from the first chamber to the second chamber of the chip where they undergo hybridization of the probes to the target amplicons.

Examples of such chips are described in the following patent documents, and the skilled reader will readily be able to make such a chip from these: FR 1043770, GB 1043770, IT 1043770, FR 1049157, GB 1049157, DE 1123739, IT 1123739, GB 1123739, FR 1123739, IT 1161985, GB 1161985, FR 1161985, FR 1182602, DE 1182602, IT 1182602, FR 1193214, IT 1193214, GB 1193214, US 6376291, US 6504226, US 6670257, US 20010024820, US 20010049200, US 20020017660, US 20030057199, US 20020045244, US 20030119289, US 20040206749, US 20040226908, US 20040235149, US 20040106290, US 20040164068, US 20020097900, US 20050136598, US 20050181392, US 20050282221, US 20050155860, US 20040141856, DE 1161985, DE 1193214, DE 1043770, DE 1049157, EP 1130631, EP 1326279, EP 1400600, EP 1403383, EP 1452993, EP 1535665, EP 1535878, EP 1541991, EP 1547688, EP 1618955, EP 1764418, EP 1885646, EP 1914226, EP 2051935, CN 101200447, WO 2006120221, JP 2006237643, WO 2007091280, WO 2007091281, WO 2007141811, US 20040132059, US 20040191804, US 20040227207, US 20050142597, US 20050176037, US 20050233440, US 20060115828, US 20070125650, US 20070252224, US 20080118405 and US 20080138210.

### Reverse Transcription and One Step RT-PCR

An influenza viral assay (or an assay of any RNA virus) may involve reverse transcription (RT) as a first step of a detection reaction. During reverse transcription RNA sequences are converted to complementary DNA (cDNA), providing a cDNA template for PCR amplification.

An approach to RT-PCR is the use of a "One-Step RT-PCR system." In a system of this type, reagents for both RT and PCR may be added to a sample in a single mixture and loaded into a chip, for example comprising probe sequences. The RT and PCR enzyme-catalyzed reactions may be carried out sequentially in the PCR micro reactor of the chip, taking advantage of the different thermostabilities of the enzymes involved (typically, a thermolabile reverse transcriptase and a thermostable DNA polymerase) and by setting an appropriate thermal profile. An initial incubation at non-denaturing temperature allows RT to occur first. The temperature then can be raised to initiate PCR.

The temperature and duration of RT and PCR steps can be readily determined by one of skill in the art. In one embodiment, an RT step can be performed for from less than 2 minutes to more than 60 minutes at a temperature of from approximately 50-60 degree. C. and a DNA-polymerase step can be performed at approximately 95 degree. C. for several cycles as discussed elsewhere.

### Controls

The methods described here may further involve the use of controls. Thus, for example, a control of non-human and non-viral may be co-amplified with the target nucleic acid.

Such a control may be used to control the integrity of reagents, equipment and the presence of inhibitors, etc. This control can be observed in all tests even in the absence of influenza. The control may be generated by amplification of an internal control target and hybridized to a complimentary probe.

Hybridization control probes may also be employed. Such hybridisation controls may be spotted onto the microarray to serve as a control to the hybridization process. Such control sequences or probes may comprise for example phytochromobilin synthase HY2 protein from *Arabidopsis thaliana.*

### Subtype and Strain Identification

Using the information obtained from an amplification reaction, various strains and sub-types of an influenza virus may be distinguished from each other and the strain and sub-type identified.

Specifically, an assay may be used to provide a positive or negative (yes/no) determination of the likely presence or absence of influenza virus types A and B as well as sub-type identification of H1, H1N1, H3, H3N2, H5, H5N1, H7, H9, H9N2 and Influenza B, etc in a sample.

An assay may also be used to monitor for one or more mutations in an influenza virus strain. Mutations in an influenza virus, within, for example the HA and NA, can alter viral infectivity and lethality in different hosts and different tissues, and may also be detected using the methods and compositions described here.

### Gene and Variant Detection

The influenza sequences described here are in particular capable of binding to a relevant influenza gene of an influenza strain.

The influenza sequences may therefore in particular be capable of binding to HA (hemagglutinin) or NA (neuraminidase) variants from different influenza strains. They may be used to target the HA gene or the NA gene or both. The influenza sequences may be such that they are capable of binding to certain gene variants and not others. The influenza sequences may be capable of binding to only one gene variant.

Thus, we disclose influenza sequences comprising nucleic acid sequences capable of binding to different HA gene variants. We disclose influenza sequences comprising nucleic acid sequences capable of binding to a H1 gene variant. We disclose influenza sequences comprising nucleic acid sequences capable of binding to a H3 gene variant. We disclose influenza sequences comprising nucleic acid sequences capable of binding to a H5 gene variant. We disclose influenza sequences comprising nucleic acid sequences capable of binding to a H7 gene variant. We disclose influenza sequences comprising nucleic acid sequences capable of binding to a H9 gene variant.

We further disclose influenza sequences comprising nucleic acid sequences capable of binding to different N gene variants. We disclose influenza sequences comprising nucleic acid sequences capable of binding to an N1 gene variant.

The influenza sequences may be used to discriminate between different strains of flu virus. For example, they may be used to discriminate between H1N1, H3N2, H5N1 and H9N2. The sequences may be used to detect the HA genes of subtypes H1N1, H3N2, H5N1, H7, H9N2 and Influenza B. The sequences may be used to detect NA genes of subtypes H1N1 and H5N1, for example. They may be used in a microarray to identify the abovementioned specific strains of human Influenza A and B.

### Multiplex Detection

The nucleic acid sequences described here may be used in a method of multiplex detection of targets.

The primers or primer sets may be designed in such a way that each primer set primes a fragment that is as short as possible. This may be done to increase hybridization efficiency and to achieve shorter PCR cycling time while flanking as many potential probe sites as possible.

The length of the primers may be about 30-mer to allow for higher reverse transcription and annealing temperature to improve specificity. The sequences of the different strains of each subtype may be aligned and potential probes sites for each subtype may be identified. Sites flanking regions containing the most number of potential probe sites may then be used to design the primers.

The methods described in this document may therefore be multiplexed. In accordance with the invention, more than one primer pair is used in combination with one or more probes to amplify and detect specific target nucleic acid sequences of influenza.

In some embodiments, target specific sequences from the influenza A and B may include the use of the primers and probes as set forth in Table D1 and D2. We provide for the use of all PCR primers in Table D1 and all the probe sequences in Table D2.

The influenza sequences may be used in any method for which nucleic acid sequences are generally suitable. They may in particular be used as primers or probes for the detection of influenza, for example as described above.

### Assay Reagents

Assay reagents, for example, comprising the primers and probes described above, may be provided as a kit or a consumable. The reagents may be supplied as a lyophilized preparation. Each reagent can be supplied separately or as a mixture of one or more reagents. Reagents also can be supplied on a substrate, such as a bead. A lyophilized reagent can be stable for more than one year.

### INFLUENZA SEQUENCES (TABLE D1)

| | **Primer name** | **Primer sequence** |
|---|---|---|
| SEQ ID NO: 1 | H1N1F_deg | 5'-GAC ACA GTA CTW GAA AAG AAT GT-3' |
| SEQ ID NO: 2 | H1N1R_deg | 5'-TTT CYA CAA TGT AGG ACC ATG A-3' |
| SEQ ID NO: 3 | NA_H1N1_F | 5'-GAA GTT CCC TCT CCA TAC AA-3' |
| SEQ ID NO: 4 | NA_H1N1_R | 5'-GTT ATT ATG CCG TTG TAC TTT-3' |
| SEQ ID NO: 5 | H3N2_873U22 | 5'-CAA AAT ACG AAG TGG GAA AAG C-3' |
| SEQ ID NO: 6 | H3N2_1207L23 | 5'-ATT TGA TTG ATT GCT GCT TGA GT-3' |
| SEQ ID NO: 7 | H5N1HA_517U21 | 5'-AAA CGG GCA AAG TGG AAG GAT-3' |
| SEQ ID NO: 8 | H5N1 HA 769L25 | 5'-CTA ATC TGT TTG ATT TCA CAT ATT T-3' |
| SEQ ID NO: 9 | H5N1NA_542U24 | 5'-ATA ATA ACA GAC ACT ATC AAG AGT-3' |
| SEQ ID NO: 10 | H5N1NA_820L24 | 5'-CTC CAA ATT TTG ATT GAA AGA TAC-3' |
| SEQ ID NO: 11 | H7_1185U23 | 5'-GCT TAT AGA GAA AAC TAA CCA AC-3' |
| SEQ ID NO: 12 | H7_1475L23 | 5'-ATC ATA AGT GTT GTT TCT AAT AC-3' |
| SEQ ID NO: 13 | H9N2_581U23 | 5'-TGA CAA CAG AAG ACA TAA ATA GG-3' |
| SEQ ID NO: 14 | H9N2_894L23 | 5'-TTG AGA CTC TTT ACT CCA ACA TA-3' |
| SEQ ID NO: 15 | FluB_743U21 | 5'-AAA CAG AAG ACG GAG GAC TAC-3' |
| SEQ ID NO: 16 | FluB_1041L22 | 5'-CAG GAG GTC TAT ATT TGG TTC C-3' |

H1N1F_deg and H1N1R_deg are sequences suitable for use as primers for the detection of H1N1 strains, including Swine Flu (H1N1) and seasonal H1N1 flu. NA_H1N1_F and NA_H1N1_R are sequences suitable for use as primers for the detection of NA of H1N1.

H3N2_873U22 and H3N2_1207L23 are sequences suitable for use as primers for the detection of H3N2.

H5N1HA_517U21 and H5N1 HA 769L25 are sequences suitable for use as primers for the detection of HA of H5N1.

H5N1NA_542U24 and H5N1NA_820L24 are sequences suitable for use as primers for the detection ofNA of H5N 1.

H7_1185U23 and H7_1475L23 are sequences suitable for use as primers for the detection of H7.

H9N2_581U23 and H9N2_894L23 are sequences suitable for use as primers for the detection of H9N2.

FluB_743U21 and FluB_1041L22 are sequences suitable for use as primers for the detection of Influenza B.

### INFLUENZA SEQUENCES (TABLE D2)

| | **Target** | **Probes** | **Sequence 5'-3'** |
|---|---|---|---|
| SEQ ID NO: 17 | HA gene of H1 and H1N1 | HA_H1N1_10H | AAATCCAGAGTGTGAATCACTCTCCA |
| SEQ ID NO: 18 | HA gene of H1 and H1N1 | HA_H1N1_11H | TTGGGTAACTGCAGCGTTGCCGGGTG |
| SEQ ID NO: 19 | HA gene of H1 and H1N1 | HA_H1N1_12H | TGGAAAACTATGTCTATTAAAAGGAA |
| SEQ ID NO: 20 | HA gene of H1 and H1N1 | HA_H1N1_13H | GTCAACCTGCTTGAGGACAGTCACAA |
| SEQ ID NO: 21 | HA gene of H1 and H1N1 | HA_H1_1 | AGAATCAACTACTACTGGACTCTGC |
| SEQ ID NO: 22 | HA gene of H1 and H1N1 | HA_H1N1_5 | ATCAGGAATCATCACCTCAAATGCA |
| SEQ ID NO: 23 | HA gene of H1 and H1N1 | HA_H1N1_6 | CTTTCCAGAATGTACACCCAGTTACA |
| SEQ ID NO: 24 | Swine HA gene of H1N1 | HA_H1N1_10 | AAATCCAGAGTGTGAATCACTCTCCA |
| SEQ ID NO: 25 | Swine HA gene of H1N1 | HA_H1N1_11 | TTGGGTAAATGTAACATTGCTGGCTG |
| SEQ ID NO: 26 | Swine HA gene of H1N1 | HA_H1N1_12 | CGGGAAACTATGCAAACTAAGAGGGG |
| SEQ ID NO: 27 | Swine HA gene of H1N1 | HA_H1N1_13 | GTTAACCTTCTAGAAGACAAGCATAA |
| SEQ ID NO: 28 | Swine NA gene of H1N1 | NA_H1N1_1 | GATTTGAGTCAGTCGCTTGGT |
| SEQ ID NO: 29 | Swine NA gene of H1N1 | NA_H1N1_2 | TGCTTGTCATGATGGCATCAAT |
| SEQ ID NO: 30 | Swine NA gene of H1N1 | NA_H1N1_3 | CAATGGGGCAGTGGCTGTGT |
| SEQ ID NO: 31 | HA gene of H3 and H3N2 | HA_H3_5 | GATGGTTGGTACGGTTTCAGGCA |
| SEQ ID NO: 32 | HA gene of H3 and H3N2 | HA_H3_7 | TGTAAACAGGATCACATATGGGGC |
| SEQ ID NO: 33 | HA gene of H3 and H3N2 | HA_H3N2_2 | AATGTACCAGAGAAACAAACTAGAG |
| SEQ ID NO: 34 | HA gene of H3 and H3N2 | HA_H3N2_5 | AATGAGATCAGATGCACCCATTGGC |
| SEQ ID NO: 35 | HA gene of H5 and H5N1 | HA_H5_2 | TCAGCAATTATGAAAAGTGAATTGG |
| SEQ ID NO: 36 | HA gene of H5 and H5N1 | HA_H5_9 | ACACCAAGTGTCARACTCCAATGGG |
| SEQ ID NO: 37 | HA gene of H5 and H5N1 | HA_H5N1_6 | GAGAGTAATGGAAATTTCATTGCTC |
| SEQ ID NO: 38 | HA gene of H5 and H5N1 | HA_H5N1_9 | GAGAGTAATGGAAATTTCATTGCTCCAG |
| SEQ ID NO: 39 | NA gene of N1 and H5N1 | NA_N1_H274Y | CTAATTATTACTATGAGGAATGCTC |
| SEQ ID NO: 40 | NA gene of N1 and H5N1 | NA_N1_I222T_2 | AACAACACACTGAGAACTCAAGAGT |
| SEQ ID NO: 41 | NA gene of N1 and H5N1 | NA_H5N1_8 | GCTCCTAATTATCACTATGAGGAAT |
| SEQ ID NO: 42 | NA gene of N1 and H5N1 | NA_H5N1_1 | ATATAAGATCTTCAAAATGGAAAAA |
| SEQ ID NO: 43 | HA gene of H7 | HA_H7_2 | AATGCTGAAGAAGATGGCACTGGTT |
| SEQ ID NO: 44 | HA gene of H7 | HA_H7_8 | GGCAATGTSATWAATTGGACCAGAG |
| SEQ ID NO: 45 | HA gene of H7 | HA_H7_9 | TGAGTTGATAGACAATGARTTCACTGAG |
| SEQ ID NO: 46 | HA gene of H9 and H9N2 | HA_H9_2 | GAGTAAGATCCAATGGGAATCTAAT |
| SEQ ID NO: 47 | HA gene of H9 and | HA_H9_3 | TAATTGCTCCATGGTATGGACACAT |
| SEQ ID NO: 48 | HA gene of H9 and | HA_H9_6 | AATGTCAGCAAATATGCATTTGGGA |
| SEQ ID NO: 49 | HA gene of H9 and H9N2 | HA_H9N2_5 | GGAAGAATTGATTATTATTGGTCGG |
| SEQ ID NO: 50 | HA gene of H9 and | HA_H9N2_6 | GGTGGCTTAAATACTACATGGCCAT |
| SEQ ID NO: 51 | HA gene of FluB | HA_FluB_4 | CCCAAATCAAACAGAAGACGGAGGA |
| SEQ ID NO: 52 | HA gene of FluB | HA_FluB_6 | AACAAAAGCAAGCCTTACTACACAG |
| SEQ ID NO: 53 | HA gene of FluB | HA_FluB_7 | GATTAAACAAAAGCAAGCCTTACTA |

### DETECTION USING INFLUENZA SEQUENCES

In Table D1, H1N1F_deg and H1N1R_deg are sequences suitable for use in amplifying Swine Flu H1N1 and seasonal flu H1N1. These sequences may be used in combination with, as shown in Table D2, HA_H1N1_10H for the detection of the HA gene of H 1, such as H1N1; with HA_H1N1_11H for the detection of the HA gene of H 1, such as H1N1; with HA_H1N1_12H for the detection of the HA gene of H1, such as H1N1; with HA_H1N1_13H for the detection of the HA gene of H1, such as H1N1; with HA_H1_1 for the detection of the HA gene of H1, such as H1N1; with HA_H1N1_5 for the detection of the HA gene of H 1, such as H1N1; with HA_H1N1_6 for the detection of the HA gene of H 1, such as H1N1; with HA_H1N1_10 for the detection of the Swine HA gene of H1N1; with HA_H1N1_11 for the detection of the Swine HA gene of H1N1; with HA_H1N1_12 for the detection of the Swine HA gene of H1N1; with HA_H1N1_13 for the detection of the Swine HA gene of H1N1; with NA_H1N1_1 for the detection of the Swine NA gene of H1N1; with NA_H1N1_2 for the detection of the Swine NA gene of H1N1; and/or with NA_H1N1_3 for the detection of the Swine NA gene of H1N1.

In Table D1, NA_H1N1_F and NA_H1N1_R are sequences suitable for use in amplifying NA of H1N1. These sequences may be used in combination with, as shown in Table D2, HA_H1N1_10H for the detection of the HA gene of H1, such as H1N1; with HA_H1N1_11H for the detection of the HA gene of H1, such as H1N1; with HA_H1N1_12H for the detection of the HA gene of H1, such as H1N1; with HA_H1N1_13H for the detection of the HA gene of H1, such as H1N1; with HA_H1_1 for the detection of the HA gene of H1, such as H1N1; with HA_H1N1_5 for the detection of the HA gene of H1, such as H1N1; with HA_H1N1_6 for the detection of the HA gene of H1, such as H1N1; with HA_H1N1_10 for the detection of the Swine HA gene of H1N1; with HA_H1N1_11 for the detection of the Swine HA gene of H1N1; with HA_H1N1_12 for the detection of the Swine HA gene of H1N1; with HA_H1N1_13 for the detection of the Swine HA gene of H1N1; with NA_H1N1_1 for the detection of the Swine NA gene of H1N1; with NA_H1N1_2 for the detection of the Swine NA gene of H1N1; and/or with NA_H1N1_3 for the detection of the Swine NA gene of H1N1.

In Table D1, H3N2_873U22 and H3N2_1207L23 are sequences suitable for use in amplifying H3N2. These sequences may be used in combination with, as shown in Table D2, HA_H3_5 for the detection of the HA gene of H3, such as H3N2; with HA_H3_7 for the detection of the HA gene of H3, such as H3N2; with HA_H3N2_2 for the detection of the HA gene of H3, such as H3N2; and/or with HA_H3N2_5 for the detection of the HA gene of H3, such as H3N2.

In Table D1, H5N1HA_517U21 and H5N1 HA 769L25 are sequences suitable for use in amplifying HA of H5N1. These sequences may be used in combination with, as shown in Table D2, HA_H5_2 for the detection of the HA gene of H5, such as H5N1; with HA_H5_9 for the detection of the HA gene of H5, such as H5N1; with HA_H5N1_6 for the detection of the HA gene of H5, such as H5N1; with HA_H5N1_9 for the detection of the HA gene of H5, such as H5N1; with NA_N1_H274Y for the detection of the NA gene of N1, such as H5N1; with NA_N1_I222T_2 for the detection of the NA gene of N1, such as H5N1; with NA_H5N1_8 for the detection of the NA gene of N1, such as H5N1; and/or with NA_H5N1_1 for the detection of the NA gene of N 1, such as H5N1.

In Table D1, H5N1NA_542U24 and H5N1NA_820L24 are sequences suitable for use in amplifying NA of H5N1. These sequences may be used in combination with, as shown in Table D2, HA_H5_2 for the detection of the HA gene of H5, such as H5N1; with HA_H5_9 for the detection of the HA gene of H5, such as H5N1; with HA_H5N1_6 for the detection of the HA gene of H5, such as H5N1; with HA_H5N1_9 for the detection of the HA gene of H5, such as H5N1; with NA_N1_H274Y for the detection of the NA gene of N1, such as H5N1; with NA_N1_I222T_2 for the detection of the NA gene of N1, such as H5N1; with NA_H5N1_8 for the detection of the NA gene of N1, such as H5N1; and/or with NA_H5N1_1 for the detection of the NA gene of N1, such as H5N1.

In Table D1, H7_1185U23 and H7_1475L23 are sequences suitable for use in amplifying H7. These sequences may be used in combination with, as shown in Table D2, HA_H7_2 for the detection of the HA gene of H7; with HA_H7_8 for the detection of the HA gene of H7; and/or with HA_H7_9 for the detection of the HA gene of H7.

In Table D1, H9N2_581U23 and H9N2_894L23 are sequences suitable for use in amplifying H9N2. These sequences may be used in combination with, as shown in Table D2, HA_H9_2 for the detection of the HA gene of H9, such as H9N2; with HA_H9_3 for the detection of the HA gene of H9, such as H9N2; with HA_H9_6 for the detection of the HA gene of H9, such as H9N2; with HA_H9N2_5 for the detection of the HA gene of H9, such as H9N2; and/or with HA_H9N2_6 for the detection of the HA gene of H9, such as H9N2.

In Table D1, FluB_743U21 and FluB_1041L22 are sequences suitable for use in amplifying Influenza B. These sequences may be used in combination with, as shown in Table D2, HA_FluB_4 for the detection of the HA gene of FluB; with HA_FluB_6 for the detection of the HA gene of FluB; and/or with HA_FluB_7 for the detection of the HA gene of FluB.

### OTHER INFLUENZA SEQUENCES (TABLE D3)

| **Gene** | **Primer Name** | **Sequence** |
|---|---|---|
| H1N1 HA gene | H1N12 HA F | 5'- TTC GAA ATA TTC CCC AAA GRR AGC TCA TGG - 3' |
| H1N1 HA gene | H1N1 HA R | 5'- TGC ACT CCT GAC ATA CTT TGG ACA CTC TCC- 3' |
| H3N2 HA gene | H3N2 HA F | 5'- GAA GAA TCA CAG TCT CTA CCA AAA GAA GCC- 3' |
| H3N2 HA gene | H3N2 HA R | 5'- CTC GTT GGT TTT CCC GAT CAA CCT ATT CAG- 3' |
| H5N1 HA gene | H5N1 HA F | 5'-GGT CTT ACA TAG TGG AGA AGR YCA ATC CAG- 3' |
| H5N1 HA gene | H5N1 HA R | 5'- AGG GCT ATT TCT GAG CCC AGT CGC AAG GAC- 3' |
| H5N1 NA gene | H5N1 NA F | 5'- GCT CCC TCC CCA TAT AAC TCA AGG TTT GAG - 3' |
| H5N1 NA gene | H5N1 NA R | 5'- CTC CAC TGC ATA TAT ATC CTA TTT GAT ACT CC- 3' |
| H7N1 HA gene | H7 HA F | 5'- AAC AGG AAT GAA GAA TGT TCC YGA RAT YCC- 3' |
| H7N1 HA gene | H7 HA R | 5'- TAG AAG TAT AAA ACA TGA TGC CCC GAA GCT- 3' |
| H9N2 HA gene | H9N2 HA F | 5'- CAT GTT CAG ATT CAT TCT ACA GGA GTA TGA G - 3' |
| H9N2 HA gene | H9N2 HA R | 5'- CCC TCT ACT TGA TCT AGC AGG CAC ATT CCT- 3' |
| Influenza B HA gene | Inf B HA F | 5'- AAA TTG GAA CCT CAG GGT CTT GCC CTA ACG - 3' |
| Influenza B HA gene | Inf B HA R | 5'- GGG TGT TTT CAC CCA TAT TGG GCA ATT TCC- 3' |

Table D3. Influenza sequences useful for example as primers for amplification of influenza genes.

### OTHER INFLUENZA SEQUENCES (TABLE D4)

| **Probe ID Name** | **Structure/Sequence Probe** | **Target Gene** |
|---|---|---|
| **HA_H1_4** | AGCAGTTTTTACAGAAATTTGCTAT | **H1** |
| **HA_H3_2** | **GCA**GAATAAGCATCTATTGGACAATAGT | **H3** |
| **HA_H3_3** | **TGG**GAAAAGCTCAATAATGAGATCAGAT | **H3** |
| **HA_H3_4** | CAAATGGAAGCATTCCCAATGACAA | **H3** |
| **HA_H3_6** | GTTAAGCAAAACACTCTGAAATTGGCA | **H3** |
| **HA_H5_1** | AGGTCCTCCTTTTTTAGAAATGTGG | **H5** |
| **HA_H5_7** | CCATTTTGAGAAAATTCAGATMATCCC | **H5** |
| **HA_H5_8** | AAGCCTCATCAGGRGTGAGCTCAGC | **H5** |
| **HA_H5_10** | ACGGGCAAAGTGGAAGRATGGAGTT | **H5** |
| **HA_H7_1** | ATAATGCTGAACTCTTGGTAGCAAT | **H7** |
| **HA_H7_4** | AATAGAATACAGATTGACCCAGTCA | **H7** |
| **HA_H7_7** | CCAGTCGGCAATTGATCAGATAACC | **H7** |
| **HA_H9_4** | ATATTCTHTTCATGTGGGGCATACA | **H9** |
| **HA_H9_5** | TTCAAGACGCCCAATACACAAATAAT | **H9** |
| **HA_H9_7** | TGATAGGGCCAAGGCCACTTGTCAA | **H9** |
| **HA_H1N1_2** | CATCTATCATACAGAAAATGCTTAT | **H1N1 HA** |
| **HA_H1N1_4** | ACA A(R)G AGA AAG AAG TCC TTG TGC T | **H1N1 HA** |
| **HA_H1N1_6** | CTTTCCAGAATGTACACCCAGTTACA | **H1N1 HA** |
| **HA_H1N1_7** | CAATAATATTTGAGGCAAATGGAAA | **H1N1 HA** |
| **HA_H3N2_3** | GAGACATACTTTTGATTAACAGCAC | **H3N2 HA** |
| **HA_HSN1_3** | GGCTTATCAAAAAGAACAGTACATA | **H5N1 HA** |
| **HA_H5N1_4** | GCAGACTAGGCTCTATCAAAACCCA | **H5N1 HA** |
| **HA_H5N1_5** | ACTATGAAGAACTGAAACACCTATT | **H5N1 HA** |
| **HA_H5N1_7** | CCAGAGATTGGTACCAAAAATAGCT | **H5N1 HA** |
| **HA_H5N1_8** | GACTATGAAGAACTGAAACACCTATTGAGC | **H5N1 HA** |
| **HA_H5N1_10** | TCA AGG AGA GAG AAG AAG AAG AAA G | **H5N1 HA** |
| **HA_H9N2_1** | AAGATATAAATAGAACCTTCAAACC | **H9N2 HA** |
| **HA_H9N2_3** | ATTTAAAAGGTGGTAATTGTGTAGT | **H9N2 HA** |
| **HA-H9N2_7** | TGTACACAAGGACTGACACAACAACA | **H9N2 HA** |
| **NA_N1_3** | ACTATCAAGAGTTGGAGAAACAACA | **N1 gene** |
| **NA_H5N1_5** | TGTTGCTTGGTCAGCAAGTGCTTGC | **H5N1 NA gene** |
| **NA_H5N1_7** | TGTTGCTTGGTCAGCAAGTGCTTGC | **H5N1 NA gene** |
| **HA_FluB_3** | CCCAAATCAACAGAAGACGGAGGAC | **Flu B HA gene** |
| **HA_FluB_5** | AAC GGA GTG ACC ACA CAT TAC GTT T | **Flu B HA gene** |
| **HA_FluB_8** | AAG TGG CAG GAG CAA GGT AAT AAA A | **Flu B HA gene** |

Table D4. Influenza sequences useful for example as probes for detection of different influenza strains.

### POLYNUCLEOTIDES

The influenza sequences may comprise polynucleotides. Where the context admits, the term "influenza sequence" should be taken to comprise any specific sequence disclosed in this document, for example, a sequence set out in Table D1, Table D2, Table D3 or Table D4, as well as any variants, fragments, derivatives and homologues of such specific nucleic acid sequences.

As used here in this document, the terms "polynucleotide", "nucleotide", and nucleic acid are intended to be synonymous with each other. "Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

### Degeneracy

It will be understood by a skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described here to reflect the codon usage of any particular host organism in which the polypeptides are to be expressed.

### Nature of Nucleic Acids

Influenza sequences and variants, fragments, derivatives and homologues may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of this document, it is to be understood that the polynucleotides may be modified by any method available in the art. Such modifications may be carried out in order to enhance the *in vivo* activity or life span of polynucleotides of interest.

### Variants, Homologues and Derivatives

The terms "variant", "homologue" or "derivative" in relation to a nucleotide sequence include any substitution of, variation of, modification of, replacement of, deletion of or addition of one (or more) nucleic acid from or to the sequence. The variants, homologues or derivatives may code for a polypeptide having biological activity.

### Sequence Identity

As indicated above, with respect to sequence homology, there may be at least 50 or 75%, such as at least 85% or at least 90% homology to the sequences shown in the sequence listing herein. There may be at least 95%, such as at least 98%, homology. In accordance with the invention, the variants, homologues, derivatives or fragments of the primers and probes of SEQ ID NOs: 1 to 53 have at least 90% sequence identity to the sequences given in Tables D 1 and D2. Nucleotide homology comparisons may be conducted as described above. A sequence comparison program which may be used may comprise the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

### Hybridisation

We further describe nucleotide sequences that are capable of hybridising selectively to the sequences presented herein, or any variant, fragment or derivative thereof, or to the complement of any of the above. Nucleotide sequences may be at least 15 nucleotides in length, such as at least 20, 30, 40 or 50 nucleotides in length. In accordance with the invention, the variants, homologues, derivatives or fragments of the primers and probes of SEQ ID NOs: 1 to 53 are at least 20 nucleotides in length.

The term "hybridization" as used herein may include "the process by which a strand of nucleic acid joins with a complementary strand through base pairing" as well as the process of amplification as carried out in polymerase chain reaction technologies.

Polynucleotides capable of selectively hybridising to the nucleotide sequences presented herein, or to their complement, will be generally at least 70%, such as at least 80 or 90% for example at least 95% or 98% homologous to the corresponding nucleotide sequences presented herein over a region of at least 20, such as at least 25 or 30, for instance at least 40, 60 or 100 or more contiguous nucleotides.

The term "hybridizable" may include "selectively hybridizable", i.e., that the polynucleotide used as a probe is used under conditions where a target polynucleotide is found to hybridize to the probe at a level significantly above background. The background hybridization may occur because of other polynucleotides present, for example, in the cDNA or genomic DNA library being screening. In this event, background implies a level of signal generated by interaction between the probe and a non-specific DNA member of the library which is less than 10 fo ld, such as less than 100 fold as intense as the specific interaction observed with the target DNA. The intensity of interaction may be measured, for example, by radio labelling the probe, e.g. with ³²_{P}.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical polynucleotide sequences while an intermediate (or low) stringency hybridization can be used to identify or detect similar or related polynucleotide sequences.

We describe nucleotide sequences that can hybridise to the influenza nucleic acids, fragments, variants, homologues or derivatives disclosed here under stringent conditions (e.g. 65°C and 0.1×SSC {1×SSC = 0.15 M NaCl, 0.015 M Na₃ Citrate pH 7.0).

Where the polynucleotide is double-stranded, both strands of the duplex, either individually or in combination, are encompassed by the methods and compositions described here. Where the polynucleotide is single-stranded, it is to be understood that the complementary sequence of that polynucleotide is also included.

### Deriving Variants

Polynucleotides which are not 100% homologous to the influenza sequences disclosed here but which are also included can be obtained in a number of ways. Other variants of the sequences may be obtained for example by probing DNA libraries made from a range of individuals, for example individuals from different populations. For example, influenza homologues may be identified from other individuals, or other species. Further recombinant influenza sequence variant nucleic acids and polypeptides may be produced by identifying corresponding positions in the homologues, and synthesising or producing the molecule as described elsewhere in this document.

In addition, other viral/bacterial, or cellular homologues of influenza sequences particularly cellular homologues found in mammalian cells (e.g. rat, mouse, bovine and primate cells), may be obtained and such homologues and fragments thereof in general will be capable of selectively hybridising to an influenza sequence nucleic acid. Such homologues may be used to design influenza sequence nucleic acids, fragments, variants and homologues. Mutagenesis may be carried out by means known in the art to produce further variety.

Sequences of influenza sequence homologues may be obtained by probing cDNA libraries made from or genomic DNA libraries from other animal or non-animal species, such as viral, microbial or fungal species, and probing such libraries with probes comprising all or part of any of the influenza sequence nucleic acids, fragments, variants and homologues, or other fragments of influenza sequence nucleic acids under conditions of medium to high stringency.

Similar considerations apply to obtaining species homologues and allelic variants of the polypeptide or nucleotide sequences disclosed here.

Variants and strain/species homologues may also be obtained using degenerate PCR which will use primers designed to target sequences within the variants and homologues encoding conserved amino acid sequences within the sequences of the influenza sequence nucleic acids. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences. It will be appreciated by the skilled person that overall nucleotide homology between sequences from distantly related organisms is likely to be very low and thus in these situations degenerate PCR may be the method of choice rather than screening libraries with labelled fragments the influenza sequence sequences.

In addition, homologous sequences may be identified by searching nucleotide and/or protein databases using search algorithms such as the BLAST suite of programs.

Alternatively, such polynucleotides may be obtained by site directed mutagenesis of characterised sequences, for example, influenza sequence nucleic acids, or variants, homologues, derivatives or fragments thereof. This may be useful where for example silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

The polynucleotides described here may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 8, 9, 10, or 15, such as at least 20, for example at least 25, 30 or 40 nucleotides in length, and are also encompassed by the term "polynucleotides" as used herein.

Polynucleotides such as a DNA polynucleotides and probes may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15 to 30 nucleotides) flanking a region of the lipid targeting sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector

Polynucleotides or primers may carry a revealing label. Suitable labels include radioisotopes such as ³²P or ³⁵S, enzyme labels, or other protein labels such as biotin. Such labels may be added to polynucleotides or primers and may be detected using by techniques known *per se*. Polynucleotides or primers or fragments thereof labelled or unlabelled may be used by a person skilled in the art in nucleic acid-based tests for detecting or sequencing polynucleotides in the human or animal body.

Such tests for detecting generally comprise bringing a biological sample containing DNA or RNA into contact with a probe comprising a polynucleotide or primer under hybridising conditions and detecting any duplex formed between the probe and nucleic acid in the sample. Such detection may be achieved using techniques such as PCR or by immobilising the probe on a solid support, removing nucleic acid in the sample which is not hybridised to the probe, and then detecting nucleic acid which has hybridised to the probe. Alternatively, the sample nucleic acid may be immobilised on a solid support, and the amount of probe bound to such a support can be detected. Suitable assay methods of this and other formats can be found in for example WO89/03891 and WO90/13667.

Tests for sequencing nucleotides, for example, the influenza sequence nucleic acids, involve bringing a biological sample containing target DNA or RNA into contact with a probe comprising a polynucleotide or primer under hybridising conditions and determining the sequence by, for example the Sanger dideoxy chain termination method (see Sambrook *et al*.).

Such a method generally comprises elongating, in the presence of suitable reagents, the primer by synthesis of a strand complementary to the target DNA or RNA and selectively terminating the elongation reaction at one or more of an A, C, G or T/U residue; allowing strand elongation and termination reaction to occur; separating out according to size the elongated products to determine the sequence of the nucleotides at which selective termination has occurred. Suitable reagents include a DNA polymerase enzyme, the deoxynucleotides dATP, dCTP, dGTP and dTTP, a buffer and ATP. Dideoxynucleotides are used for selective termination.

The influenza sequence variant polypeptide sequence may be used as a therapy in any form, such as in an isolated form. The term "isolated" means that the sequence is at least substantially free from at least one other component with which the sequence is naturally associated in nature and as found in nature. In one aspect, the sequence is in a purified form. The term "purified" means that the sequence is in a relatively pure state - e.g. at least about 90% pure, or at least about 95% pure or at least about 98% pure.

### DETECTION OF INFLUENZA VIRUS

The influenza sequences described here may be used for detection of influenza virus in a sample. An example follows.

The sample may be processed to extract nucleic acids, such as messenger RNA. The mRNA may be reverse transcribed to cDNA. The cDNA may be amplified using the influenza sequences as primers. Pairwise combinations of primers, such as comprising forward and reverse primers for a particular gene variant may be used. Any or all the sequences set out in Table D1 may be employed for bulk amplification of any influenza gene sequences present in the sample. The amplified nucleic acids may be detected by solution hybridisation using one or more of the influenza sequences as probes. The presence of binding between an influenza probe sequence and its target (i.e., an amplified influenza gene or portion thereof) as detected in the hybridisation may be used as an indicator of the presence of an influenza in the sample.

Specific strain detection of influenza strains may be performed by, for example, detecting binding between specific influenza probe sequences and their targets. This may be achieved for example by performing separate hybridisations and detecting binding of certain probe sequences and not others. Alternatively, or in addition, the influenza sequence probes may be immobilised in a spaced array to facilitate detection and discrimination of specific binding to certain probe sequences. Such methods of spotting nucleic acids on an array and array hybridisation and detection of binding are known in the art.

The methods described here may be used for detection of influenza infection in an individual. For this purpose, a sample may be taken from the individual and tested for the presence of influenza virus. Presence of the virus in the sample indicates influenza viral infection. Presence of a particular strain in the sample indicates infection by that strain.

It will be appreciated that it is not strictly necessary for intact influenza virus to be present in the sample. All that is necessary for detection is the presence of detectable (e.g., amplifiable) influenza nucleic acid in the sample.

The methods described here include therapeutic and prophylactic methods. Such methods include detection of influenza infection, optionally together with strain detection. It is envisaged to choose a suitable therapy based on the presence of infection, or knowledge of infection by a particular strain, or both. Suitable therapies include antivirals, flu vaccines, etc, as known in the art.

### EXAMPLES

The Examples describe a method of using the influenza gene sequences described in this document for detection of influenza, in particular, detection of influenza strains.

Some of the Examples make use of a combined RT-PCR and microarray hybridisation chip, referred to as the VereFlu Lab On Chip. However, other uses of influenza gene sequences described in this document for detection of influenza, in particular, detection of influenza strains will be evident to the skilled reader.

Such methods may include, for example, using the sequences as primers for nucleic acid amplification using for example PCR and other amplification methods, including RT-PCR, LCR, etc. The methods may also include using the sequences as probes, which may be optionally labelled, in any method of nucleic acid hybridisation, such as described in Maniatis *et al.* or by microarray hybridisation, etc.

### Example 1. Specific Tests for Influenza and Non-Influenza Strains (Materials and Methods)

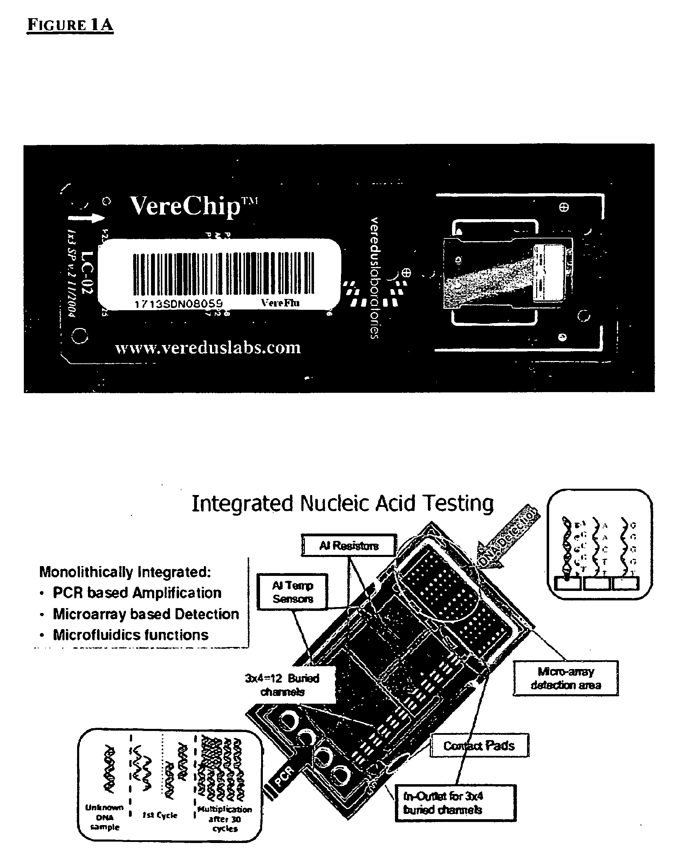

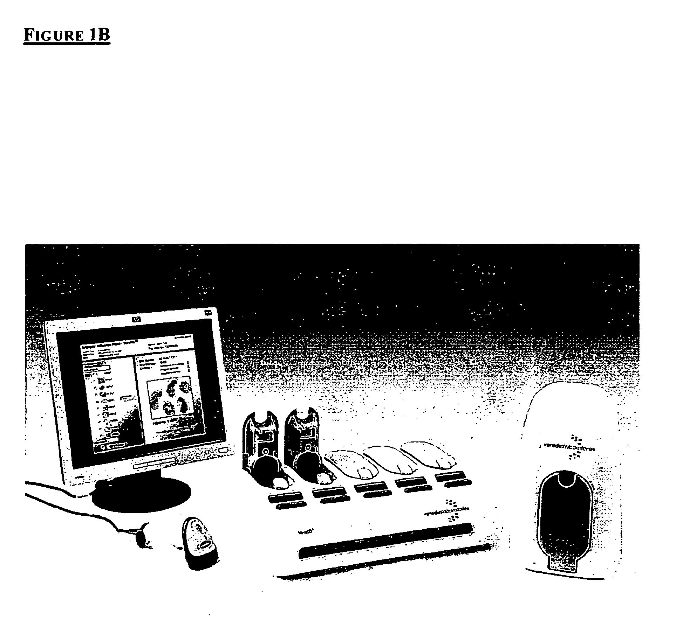

Figure 1 is a fluidic diagram that illustrates one embodiment of a Lab-on-Chip described in this document. A. VereChip- Lab-on-Chip B. VereID BioSystem.

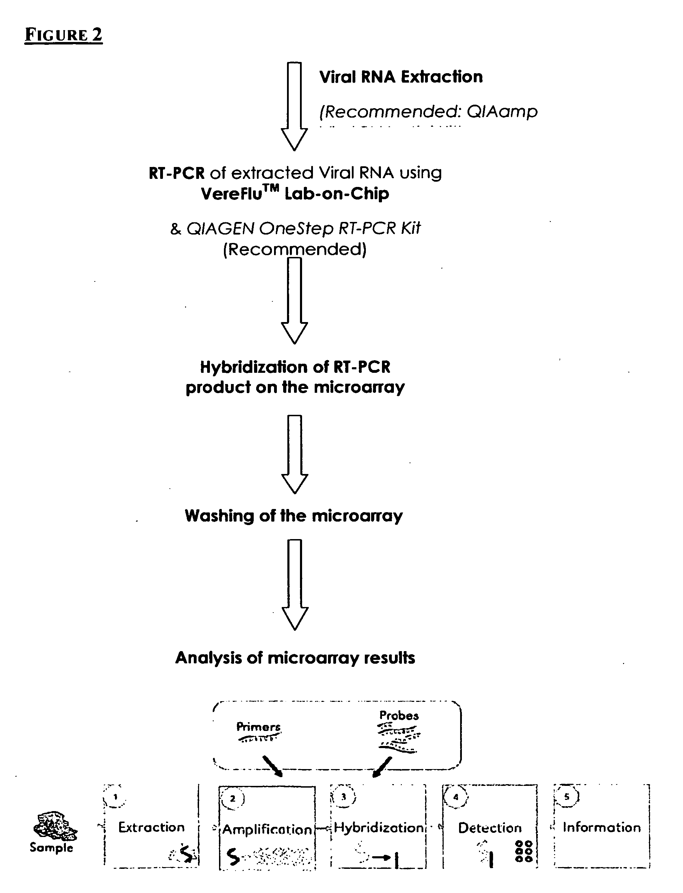

An overview of the entire protocol is depicted in Figure 2.

### Specimen Collection

Specimen collection materials adhere to the WHO guidelines for collection of Influenza viruses, as described at www.who.int/csr/disease/avian_influenza/guidelines/humanspecimens/en/index.html. Collection was done using either COPAN Universal Transport Medium (UTM) or Eagle Minimum Essential Medium (E-MEM) with veal infusion broth, bovine albumin fraction V, gentamicin sulfate solution and amphotericin B.

Collection materials are packaged with either nylon FLOCKED swab or plastic/polyester swab (commonly available non propriety specimen collection materials) together with the collection tube.

### Influenza Samples

29 RNA samples of subtypes H1N1, H3N2 and Influenza B were obtained for the experiments from clinical samples. One RNA stock sample of each subtype H7N1 and H9N2 were obtained from archived samples.

The breakdown on the number of each subtype and their origins is as follows:
i) H1N1: 5 samples, human
ii) H3N2: 13 samples, human
iii) H7N1: 1 samples, avian
iv) H9N2: 1 samples, avian
v) Inf B: 11 samples, human.

### Non-Influenza Samples

For specificity tests, five non-influenza viruses (Respiratory Syncytial Virus, Parainfluenza, Adenovirus, Metapneumovirus and Rhinovirus obtained from ATCC), with their TCID50 determined are used.

### Isolation of Viral RNA

Virus isolation is performed on patient or avian samples.

RNA is directly extracted from the specimen using a Qiagen QIAamp viral RNA extraction kit (catalog no. 52906) according to the instructions given in the product insert.

### Other Viruses

RNA is directly extracted from Respiratory syncytial virus, Human parainfluenza virus, Metapneumovirus, Adenovirus and Rhinovirus using the QIAGEN Viral RNA Mini Kit (QIAGEN GMbH, Germany) according to the instructions given in the product insert. It would be understood by those in the art that RNA isolated from any non-influenza virus would be sufficient to provide a suitable negative control.

### Reverse Transcription (RT) and Amplification by PCR

Each RNA sample (extracted using Qiagen QIAmp Viral RNA minikit, Cat no. 52904) is amplified in the integrated PCR reactor on the chip using Qiagen Quantitect virus +ROX kit (cat no. 211031). A multiplex mix of primers for the HA gene of the subtypes H1N1, H3N2, H5N1, H7N1, H9N2, Inf B and the NA gene of H5N1 are used.

The protocol used is as follows:

| ***Components*** | ***Volume (µl)*** |
|---|---|
| *Qiagen Quantitect Viral Master Mix* | *5.0* |
| *VereFlu Primer Mix A* | *1.0* |
| *RT Enzyme Mix* | *0.5* |
| *RNA* | *10.0* |
| *IVT PCR control* | *5.0* |
| *PCR-grade water* | *3.5* |
| ***Total volume*** | ***25.0*** |

### Loading

Each inlet channel of the chip is loaded with 11.5 µl of the RT-PCR mix. The chip is then placed into the Thermal Control System (TCS) of the Lab-on-Chip system, described in further detail below. RT-PCR is conducted in the PCR micro-reactor of the chip.

### RT-PCR Conditions

The cycling conditions are 50°C for 30 mins for reverse transcription; 95°C for 5 mins for enzyme activation; 45 cycles of 95°C for 15 secs, 50°C for 30 secs and 72°C for 45 secs.

### RT-PCR Product Hybridization

After RT-PCR, the RT-PCR product is displaced out of the PCR micro-reactor by pipetting 14.5 µl of hybridisation buffer into each inlet channel. Hybridisation is carried out in the TCS at 55°C for 30 min using Phosphate Buffered Solution, NaCl, KCI, Tween 20 and Denhardt's solution.

After hybridization, the microarray is washed by centrifuging it in wash buffer at 3000 rpm for 2 minutes. The wash buffer is subsequently drained and the chip placed back into the centrifuge tube to be spun-dry at 3000 rpm for 2 minutes.

### Microarray Imaging and Analysis

The hybridized microarray is scanned and the image captured using the VereID Microarray Reader. The captured image is analysed automatically by the VereID software. For a probe to be considered lit, the signal median / background median must be equal or more than 3 * max (Signal med / Background med) of the Negative Probe Controls.

Probes are pre-spotted at the microarray chamber of the chip. The probes on the chip microarray are spotted at a concentration of 10 µM.

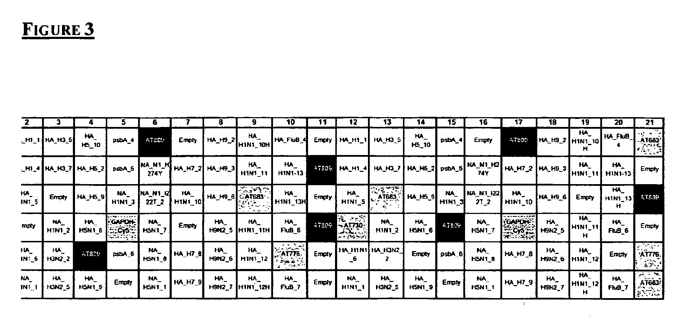

For any particular influenza type and strain, its respective probes will light up in a particular pattern on the microarray. The probe panel with predetermined and pre-spotted probes is shown in Figure 3. All probes are spotted in duplicates.

### Example 2. Specific Tests for Influenza and Non-Influenza Strains (Results)

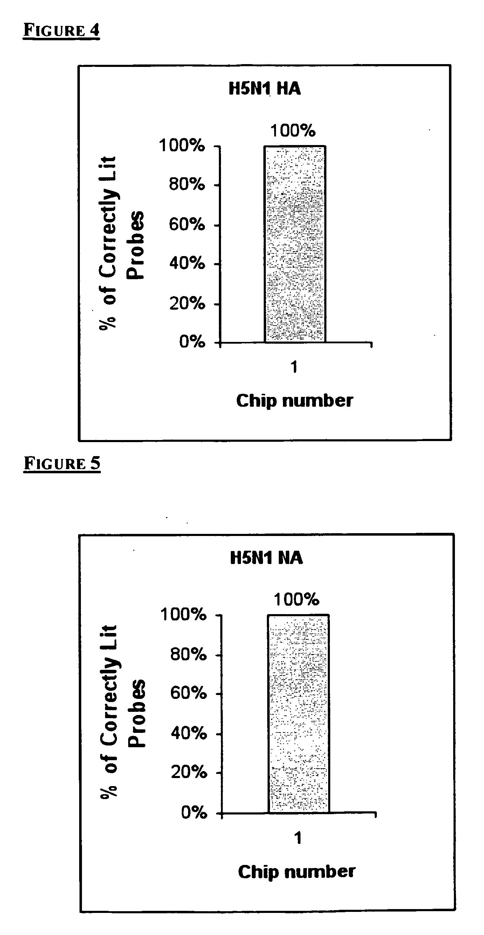

The results of correct typing of H5N1, H7, H9N2 and Inf B are shown in Figure 4, Figure 5, Figure 6 and Figure 7. These are described in further detail in Examples 3 to 6 below.

To interpret the results, a positive result with the correct typing/ subtyping of influenza is defined as the least number of probes (in duplicates) that constitutes at least 50% of the total number of probes for a particular target panel that is lit. All the samples tested produced clear identification of the correct typing.

### Example 3. Specific Tests for Influenza and Non-Influenza Strains (H5N1 HA Results)

One *in vitro* H5N1 HA RNA transcripts from its cloned gene from a single origin is used to determine the specificity of the probes on the microarray panel.

All the hybridization probes are correctly lit and no non-specific probes in the panel are lit in all chips.

A summary of the probe results can be found in Figure 4. Figure 4 shows the specificity of the probes on the microarray panel for the HA gene of the H5 type/ H5N1 subtype of influenza A.

Based on the tests done on the RNA sample, we conclude that this sub probe panel is specific for the H5 type/ H5N1 subtype of influenza A. In view of the limited number of samples for H5N1, further experiments are done to statistically verify the specificity when more samples are obtained.

### Example 4. Specific Tests for Influenza and Non-Influenza Strains (H5N1 NA Results)

One *in vitro* H5N1 NA RNA transcripts from its cloned gene from a single is used to determine the specificity of the probes on the microarray panel.

All the hybridization probes are correctly lit and no non-specific probes in the panel are lit in all chips.

A summary of the probe results can be found in Figure 5. Figure 5 shows the specificity of the probes on the microarray panel for the NA gene of the H5 type/ H5N1 subtype of influenza A.

Based on the tests done on the RNA sample, we conclude that this sub probe panel is specific for the H5 type/ H5N1 subtype of influenza A. In view of the limited number of samples for H5N1, further experiments are done to statistically verify the specificity when more samples are obtained.

### Example 5. Specific Tests for Influenza and Non-Influenza Strains (H7 HA Results)

Four different H7N 1 RNA samples are used to determine the specificity of the probes on the microarray panel.

All the hybridization probes are correctly lit and no non-specific probes in the panel are lit in both chips. The percentage of correctly lit probes are 100% (2/2), indicating a correct result for the sample.

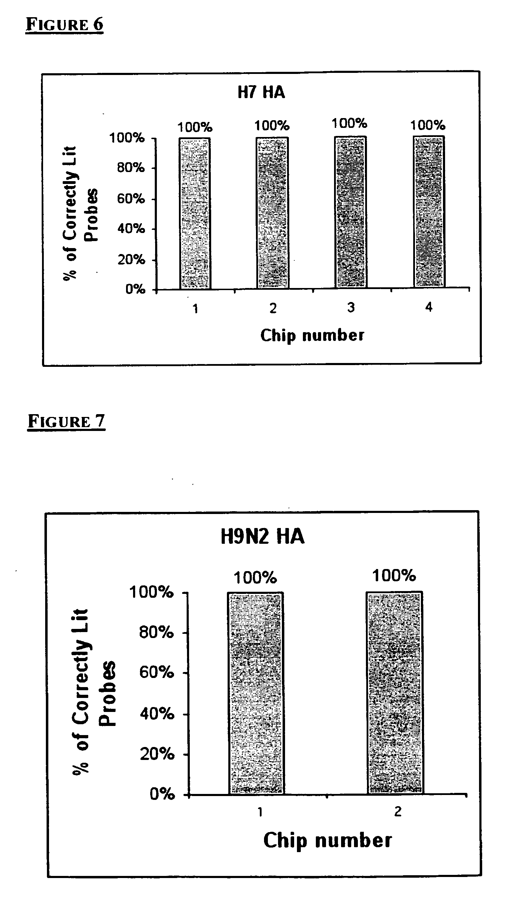

A summary of the probe results can be found in Figure 6. Figure 6 shows the specificity of the probes on the microarray panel for H7 type of influenza A.

Based on the tests done on the 4 different RNA samples, we conclude that this sub probe panel is specific for the H7 type of influenza A.

### Example 6. Specific Tests for Influenza and Non-Influenza Strains (H9N2 HA Results)

Two different H9N2 RNA samples are used to determine the specificity of the probes on the microarray panel.

In both chips, the three type/ subtype specific probes are correctly lit. All the hybridization probes are correctly lit and no non-specific probes in the panel are lit in both chips. The percentage of correctly lit probes are 100% (3/3), indicating a correct result for the sample. A summary of the probe results can be found in Figure 7. Based on the tests done on the 2 different RNA samples, we conclude that this sub probe panel is specific for the H9 type/ H9N2 subtype of influenza A.

### Example 7. Cross Reactivity Tests with Non-Influenza Viruses

Cross reactivity tests among the Influenza subtypes and with a panel of non-influenza respiratory pathogens are performed.

The influenza samples are tested at concentration of 10 x 10⁶ copies/reaction. The 5 non-influenza respiratory viruses are either obtained from the National University Hospital, Singapore or purchased from ATCC.

The results are shown in Table E4 below.

| (-) Results Negative for Reactivity | | | |
|---|---|---|---|
| (+) Results Positive for Reactivity | | | |
| ORGANISM | STRAIN | FluA | FluB |
| Influenza A virus | H1N1 | (+) | (-) |
| | H3N2 | (+) | (-) |
| | H5N1 | (+) | (-) |
| | H7N1 | (+) | (-) |
| | H9N2 | (+) | (-) |
| Influenza B virus | | (-) | (+) |
| Adenovirus | | (-) | (-) |
| Human parainfluenza virus | | (-) | (-) |
| Respiratory syncytial virus | | (-) | (-) |
| Metapneumovirus | | (-) | (-) |
| Rhinovirus | | (-) | (-) |

Table E4. No cross-reactivity of the probes on the microarray panel with non-influenza viruses.

Table E4 shows that the VereFlu primers and probes sets don't cross-react with any non-influenza viruses and also the specificity among the different Flu types/subtypes is 100%.

### Example 8 Sensitivity Tests for Influenza (Limit of Detection)

The assay limit of detection and cutoff was determined for influenza A type H1N1, H3N2, H5N 1 and influenza B. No reference materials are used at this stage of the product development.

In-vitro transcribed RNA (IVT-RNA) of the HA gene for H3N2 and IVT-RNA of HA gene and NA gene for H5N1 were used. Quantified (TCID50/ml) viral culture for Influenza B was used in this study. The IVT-RNA samples were quantified by Agilent bioanalyzer (Kit RNA 6000 Nano), then 100-fold dilutions from 10e7 to 10 copies/reaction of the IVT-RNA samples were analyzed by simplex real-time RT-PCR using VereFlu specific primers to check their functionality. Flu B RNA extracted from viral culture was quantified by real-time RT-PCR using the IVT-RNA samples previously quantified to create the standard curve.

A ten-fold dilution series from H3N2, Flu B and H5N1 RNA were made and the dilutions tested to determine the dilution that yields approximately 50% positive and 50% negative results. This dilution is then tested up to 20 replicas to determine the cutoff concentration. Next, the cutoff concentration was increased 2 times and tested up to 20 replicas. The percentage of positive and negative results in these replicas were calculated to verify the LoD concentration which must yield approximately 95% positive results.

The results are summarized in Table E5 below.

**Table E5. Limit of Detection of H1N1, H3N2, H5N1 and Influenza B.**

| **Sample type tested** | **LoD/CUTOFF** | **Performance Results** |
|---|---|---|
| H1N1 RNA | LoD | 10e2 TCID₅₀/ml |
| H3N2 HA IVT-RNA | LoD | 2x10² copies/reaction |
| | Cutoff | 10² copies/reaction |
| H5N1 HA+NA IVT-RNA | LoD | 2x10² coipes/reaction |
| | Cutoff | 10² copies/reaction |
| Influenza B virus RNA | LoD | 10² TCID₅₀/ml |
| | Cutoff | 50 TCID50/ml |

### Example 9. Clinical Trial with Patient Samples for Sensitivity Determination

RNA is directly extracted from throat swabs obtained from patients. RNA is split into 2 sets - one for real time PCR and sequencing and the other for use on VereFlu.

A total of 29 samples are extracted and identified by real time PCR and sequencing.

The number of samples and sample type is listed in Table E6.

**Table E6. Results of trial with samples of H1, H3 and Influenza B.**

| **Samples Type** | **Virus Culture"Gold standard" results** | **VereFlu Results** | **Agreement** |
|---|---|---|---|
| 29 patients samples | H1 N475 | H1N1 | yes |
| | H1 N507 | H1N1 | yes |
| | H3 N463 | H3N2 | yes |
| | H3 N534 | H3N2 | yes |
| | H3 N530 | H3N2 | yes |
| | H3 N541 | H3N2 | yes |
| | FluB N113 | Flub | yes |
| | FluB N133 | Flub | yes |
| | FluB N223 | Flub | yes |
| | H1 N157 | H1N1 | Yes |
| | H1 N163 | H1N1 | Yes |
| | H3 N185 | H3N2 | Yes |
| | H3 N105 | H3N2 | Yes |
| | H3 N116 | H3N2 | Yes |
| | H3 N125 | H3N2 | Yes |
| | H3 N127 | H3N2 | Yes |
| | H3 N 196 | H3N2 | Yes |
| | FluB N133 | FluB | Yes |
| | FluB N167 | FluB | Yes |
| | H1 N196 | Negative | No |
| | H3 N 193 | H3N2 | Yes |
| | H3 N201 | H3N2 | Yes |
| | Negative N123 | H3N2 | No |
| | FluB N173 | FluB | yes |
| | FluB N431 | FluB | Yes |
| | FluB N435 | FluB | Yes |
| | FluB N384 | FluB | Yes |
| | FluB N292 | Negative | No |
| | FluB N416 | Negative | No |

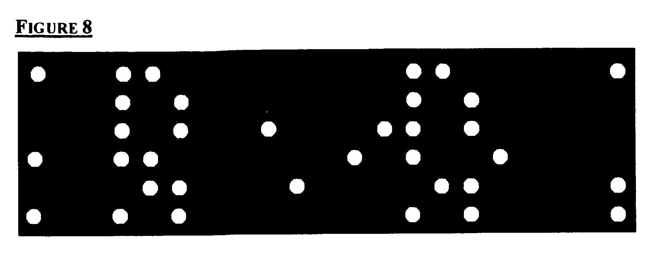

A representative of the microarray images showing H5N1 is depicted in Figure 8.

### Example 10. Clinical Sensitivity Trial Results

A summary of the results can be found in Table E1 below for H1 type/ H1N1 subtype of the influenza A.

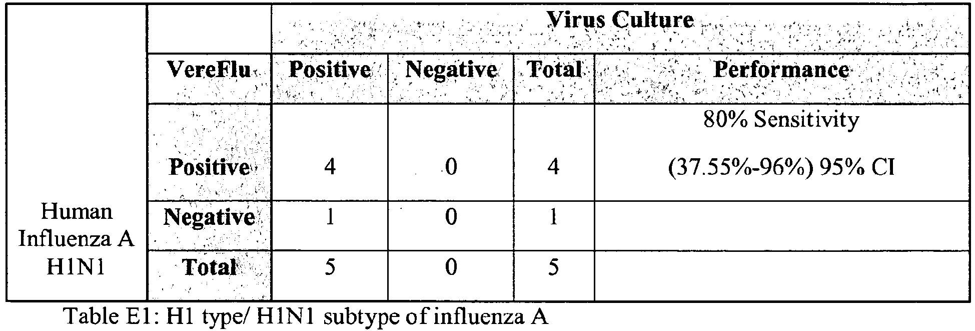

A summary of the results can be found in Table E2 below for H3 type/ H3N2 subtype of the influenza A.

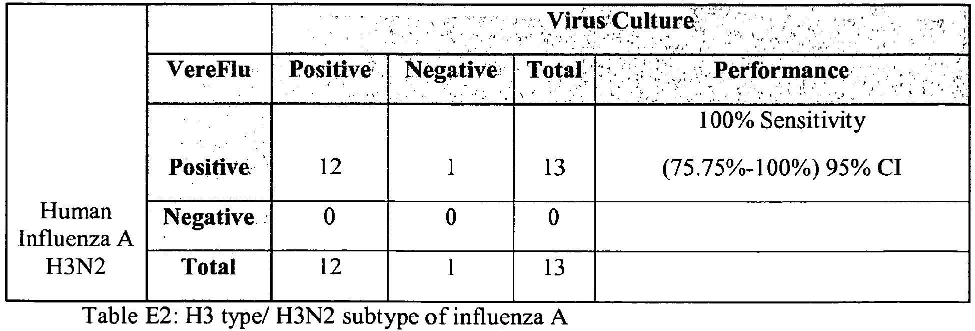

A summary of the results can be found in Table E3 below for Influenza B.

**Table E3: Influenza B**

| | | **Virus Culture** | | | |
|---|---|---|---|---|---|
| | **VereFlu** | **Positive** | **Negative** | **Total** | **Performance** |
| | | | | | 81.8% Sensitivity |
| | **Positive** | 9 | 0 | 9 | (52.36%-94.8%) 95% CI |
| | **Negative** | 2 | 0 | 2 | |
| Influenza B | **Total** | 11 | 0 | 11 | |

### Examples 11 to 18. Detailed Examples

The following Examples 11 to 18 describe a specific method of using the influenza gene sequences described in this document for detection of influenza, in particular, detection of influenza strains.

The Examples make use of a combined RT-PCR and microarray hybridisation chip, referred to as the VereFlu Lab On Chip. However, other uses of influenza gene sequences described in this document for detection of influenza, in particular, detection of influenza strains will be evident to the skilled reader.

Such methods may include, for example, using the sequences as primers for nucleic acid amplification using for example PCR and other amplification methods, including RT-PCR, LCR, etc. The methods may also include using the sequences as probes, which may be optionally labelled, in any method of nucleic acid hybridisation, such as described in Maniatis *et al.* or by microarray hybridisation, etc.

### Example 11. Preliminary Preparation, Precautions and Kit Contents

Before using this kit, please carry out the following:
- Check the content of the kit
- Spin down all tubes provided
- DILUTE the VereFlu^{™} Primer Mix provided

### Preparation of RNA Samples

While it is optional, the addition of RNase inhibitor into the RNA sample to prevent RNA sample degradation is highly recommended.

It is recommended that RNase Inhibitor be added to the RNA samples immediately after their preparation. If this is not possible, it can be added in the PCR Master Mix.

### Kit Contents: VereFlu^{™} Influenza A/B Detection Kit (Catalog no.: VFLUA/B)

### a) Cold Items:

| **VereFlu™ Influenza A/B Detection Kit (Reagents)** | | 50 reactions |
|---|---|---|
| **Part No.** | **Description** | **Quantity** |
| FLU-PMA01 | Primer Mix A | 1 tube |
| FLU-PMB01 | Primer Mix B | 1 tube |
| VCP-MHB01 | Microarray Hybridization Buffer | 2 tubes |
| VCP-CTL02 | PCR Control | 1 tube |

### b) Room Temperature Items:

| **VereFlu™ Influenza A/B Detection Kit (Detection Chips)** | | 50 reactions |
|---|---|---|
| **Part No.** | **Description** | **Quantity** |
| FLU-33 | VereFlu™ Detection Chips (25 Chips) | 2 boxes |
| COT-01 | PCR Clamps (55 outlet clamps) | 1 bag |
| CIN-01 | IN Clamps (55 inlet clamps) | 2 bags |
| CHY-01 | Hyb Clamps (55 hybridization clamps) | 1 bag |
| VCP-WBC01 | Wash Buffer Concentrate, 150 ml | 1 bottle |
| - | Empty Bottle for Dilution of Wash Buffer (1 L) | 1 bottle |

### Additional Materials and Devices Required but not Provided

- QIAamp Viral RNA Mini Kit, Qiagen (Cat. No. 52904) or equivalent
- Quantitect Virus + ROX Vial Kit, Qiagen (Cat. No. 211031)
- RNase Inhibitor (Recommended: Promega Cat. No. N2111)
- Disposable powder-free gloves
- Micropipettes (adjustable) and respective sterile filtered tips
- Sterile filtered tips, Axygen (Cat No. TF-100-L-R-S)
- Sterile 50 ml Centrifuge Tubes (non-skirted)
- Centrifuge with rotor or adapter for 50 ml Centrifuge Tube (non-skirted)
- Ice or cooler unit

### Example 12. Storage Conditions

All items of the VereFluTM Influenza A/B Detection Kit (Reagents) should be stored at -20°C and are stable until the expiry date stated on the tube label. Repeated thawing and freezing (>6x) should be avoided, as this may reduce the sensitivity of the components.

All items of the VereFluTM Influenza A/B Detection Kit (Detection Chips) should be stored at 25°C and are stable until the expiry date stated on the label. Precipitation or crystallization might occur in Wash Buffers when stored at low temperature conditions.

### Example 13. General Precautions

The user should always pay attention to the following:
- All samples should be treated as potentially infectious.
- Clinical samples from humans, birds or swine should never be processed in the same laboratory.
- Use sterile pipette tips with filters.
- Use designated pipette for each reagent if possible.
- Store and extract specimens separately from all other reagents.
- Extracted specimen should be added to the reaction mix in a separate area.
- Handle the positive control provided in the kit carefully. Do not contaminate the sample with positive control.
- Thaw all components thoroughly on ice before starting.
- After thawing, briefly centrifuge the components.
- Work quickly on ice.
- Always observe laboratory safety rules and procedures as defined by approved biohazard safety guidelines or regulations.

### Example 14. Additional Precautions when Handling RNA Samples

- The user should always pay special attention to the following:
- Always wear gloves while handling reagents and RNA samples.
- Use only sterile and RNase-free disposable plastic ware.
- RNA work should be done in a separate area.

### Example 15. Quality Control

Under Veredus' quality assurance program, the performance of our VereFluTM Influenza A/B Detection Kit is monitored routinely and on a lot-to-lot basis. Sampling is done on each lot and tests carried out via amplification of the respective control viral plasmid genes.

### Example 16. Principle of Kit Usage

Avian influenza viruses are Type A *Orthomyxoviridae* viruses that infect birds and on occasions, humans. The viruses are divided into subtypes on the basis of their surface proteins-hemagglutinin (HA) and neuraminidase (NA). Sixteen H subtypes are known. The highly pathogenic varieties are of subtypes H5 and H7. Three subtypes of HA (H1, H2, and H3) and two subtypes ofNA (N1 and N2) are known to circulate in humans.

VereFlu^{™} is a fast diagnostic test using the LOC platform to identify and differentiate all human types of Influenza A and B, with special focus on the identification of H5N1. VereFlu^{™} consists of a silicon chip integrating an ultra-fast miniaturized reactor for Reverse Transcription of RNA, PCR-based DNA amplification and a high-grade quality customised microarray for fast and accurate detection of Influenza.

By merging two robust and time-tested technologies, PCR and DNA microarray, into a single chip, we have created a test that combines the high sensitivity and specificity of the polymerase chain reaction (PCR) with the powerful identification capability of the microarray to provide answers to multiple questions within the shortest possible timeframe in a single test.

Any positive samples should be forwarded to a National Influenza Centre or a WHO Collaborating Centre for Influenza Reference and Research for further testing.

### Example 17. Protocol for VereFlu^{™} Influenza A/B Detection

**NOTE:** The following protocol is for use with Qiagen QuantiTect Virus +ROX Vial Kit only.

**NOTE:** Use Primer Mix A for detection of H1N1, H3N2, H5N1 or Influenza B. Use Primer Mix B for detection of H7 and H9N2 subtypes.

### i) Add the components in the following order into a sterile PCR tube on ice.

| **Component** | **Volume** |
|---|---|
| ***Master Mix*** | |
| 5X QuantiTect Virus +ROX Vial Kit Buffer | 5.0 µl |
| Primer Mix A (or B) | 1.0 µl |
| PCR Grade H₂O | 3.0 µl |
| RNase inhibitor (20 U/µl) | 0.5 µl ** |
| QuantiTect Virus +ROX Vial Kit Enzyme Mix | 0.5 µl |
| PCR Control | 5.0 µl |
| Template RNA | 10.0 µl |
| **Final volume** | **25 µl** |

| | |
|---|---|
| ** Optional but highly recommended | |

To prepare the PCR Master Mix for more than one reaction, multiply the amount in the "Volume" column above by the number of reactions.

**NOTE:** Always prepare 1-2 more reactions than actual number of reactions.

### Loading of the RT-PCR Mix into VereFlu^{™} Lab-On-Chip

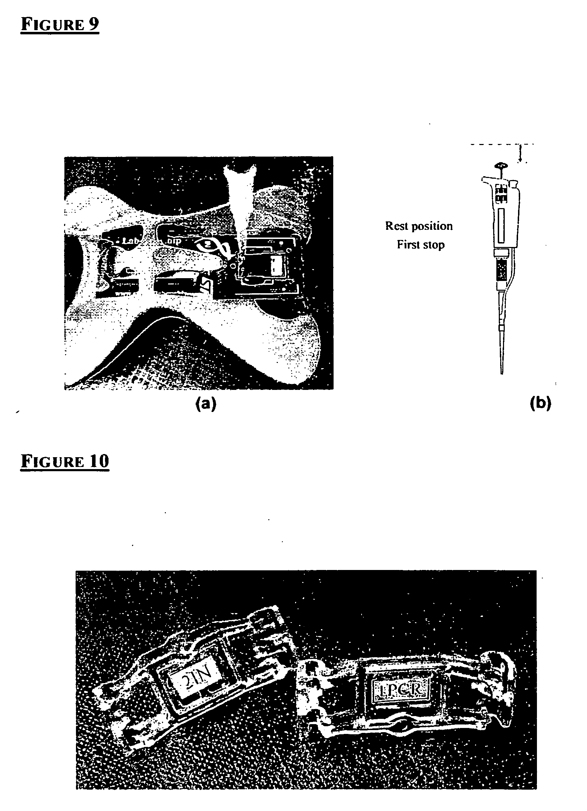

i) The following components are necessary for the loading of the RT-PCR mix into the chip:
   Clamp **IN** (labeled '2IN')
   Clamp **PCR** (labeled '1PCR')
   Clamp **HYB** (no label)
   Chip **Holder**
   Pipette and tips
ii) Insert the VereFlu^{™} Lab-On-Chip into the specific Holder to ensure a secure hold.
iii) Use a calibrated 20 µl pipette to load each inlet. The volume of the RT-PCR mix that can be dispensed is 11.5 µl for each inlet (set the pipette accordingly using the adjustment knob), thus giving an overall mix volume of 23 µl per chip.
iv) Hold the pipette in a vertical position, in such a way that the tip (currently ***Axygen** TF-100-L-R-S, Maxymum Recovery)* is perpendicular to the surface.
v) Fit the tip exactly into the inlet hole, making sure that it sits securely (see Figure 9A).
vi) Applying slight pressure onto the tip, press the plunger smoothly to the first stop position (see Figure 9B), allowing the mix to flow into the PCR chamber.
vii) Do not press the plunger beyond the first stop as this will introduce air into the chip and displace the loaded mix into the microarray well. Keep the plunger at the first stop until you remove the tip from the inlet hole (this procedure avoids spilling and the injection of air inside the chambers).

Press and release the pipette plunger slowly at all the times. Never allow the push button to snap back. Check for foreign particles in the tip. Hold the pipette in an upright position while aspirating liquid.

### Chip Sealing for RT-PCR Phase

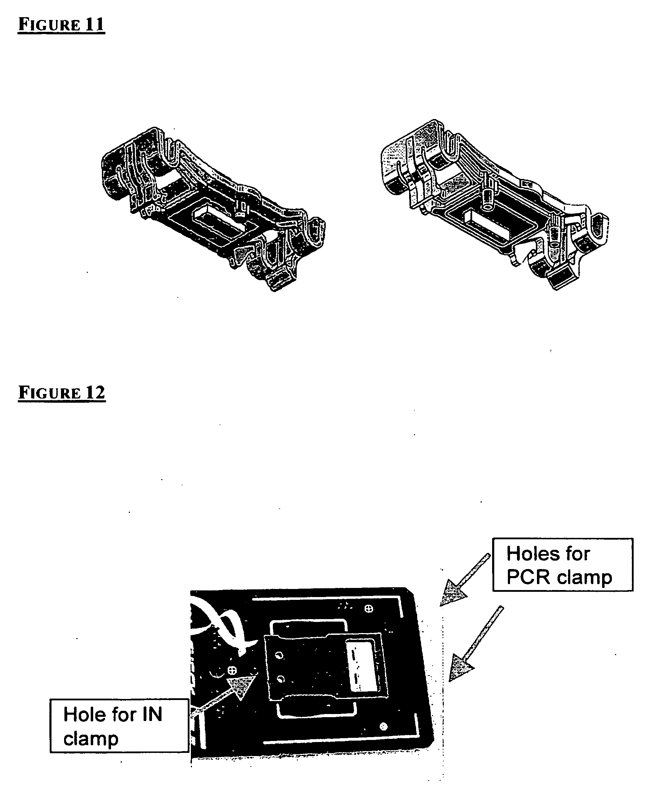

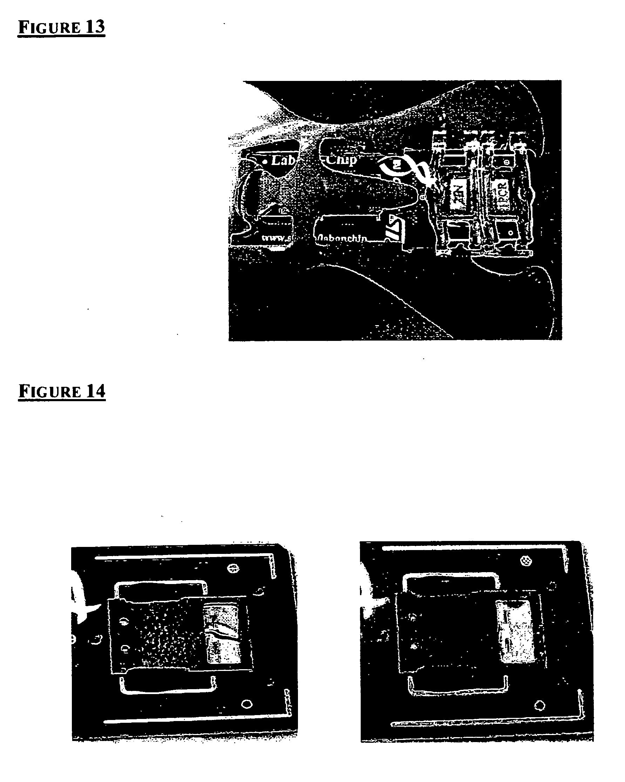

i) The IN and PCR sealing clamps are shown in Figure 10. One clamp (labeled '2IN') is dedicated to sealing the inlet holes, and the other (labeled '1PCR') to sealing the outlet holes during PCR cycles.
ii) The clamps undersides are different, owing to their specific sealing function: the IN clamp has (Figure 11, on the left) an elastomer with a rectangular protrusion of dimensions 3x7 mm (this protrusion seals the inlet holes) and one alignment pin that fits the corresponding hole on the chip (Figure 12); the PCR clamp has (Figure 12, on the right) an elastomer with a rectangular protrusion of dimensions 1.2x7 mm (this protrusion seals the outlet holes) and two alignment pins that fit the corresponding holes on the chip (Figure 12).
iii) Attach the PCR clamp first **(1PCR)**, pressing the lateral flyers and placing the pin in the alignments hole (Figure 12). After reaching the final position (the solid part of the clamp touches the edge of the chip) release the flyers and press the upper part of the clamp until a 'click' sound is heard.
iv) Attach the IN clamp (**2IN**) by proceeding in the same manner as that for the PCR clamp (Figure 12).
v) After the chip is sealed (Figure 13), remove it from the Holder and insert it into the TCM housing tray to start the thermal cycling.

Set the protocol program as follows:
**Thermal Cycling Condition:**
   **Initial Hold:**
      50°C for 20 min
   **Initial Denaturation:**
      95°C for 5 mins
      **PCR protocol:** 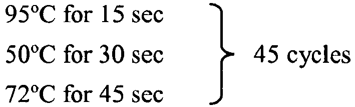
   **Denaturation:**
      95°C for 2 min
   **Hybridization:**
      55°C for 30 min

Once the final extension step of the protocol is completed, the protocol will not proceed further until the chip is set up for the hybridization process.

### Microarray Hybridization

Important Note: Thaw the Microarray Hybridization Buffer at room temperature for 15 minutes before use. Keep the Microarray Hybridization Buffer away from light until ready to use and mix gently before use in case of precipitation.
i) Remove the VereFlu^{™} Lab-On-Chip from the TCM when prompted.
ii) Insert the VereFlu^{™} Lab-On-Chip onto the specific Holder to ensure a secure fit. Remove the IN and PCR clamps and dispose them. DO NOT reuse the clamps.
iii) Use a calibrated pipette to fill each inlet with 14.5 µl of Hybridization Buffer. The total volume of hybridization buffer used for each chip is 29 µl. Use a new tip for every loading to prevent contamination.
iv) Hold the pipette in such a way that the tip is perpendicular to the surface.
v) Fit the tip exactly onto the inlet holes, ensuring that it sits in the inlet hole. Do not apply excessive pressure on the tip.
vi) Deliver the preset volume by gently pressing the plunger to the first stop position. The RT-PCR mix contained inside the channels will be displaced by the hybridization buffer and will be observed to fill up in the microarray chamber (Figure 14).

### Chip Sealing for Hybridization Phase

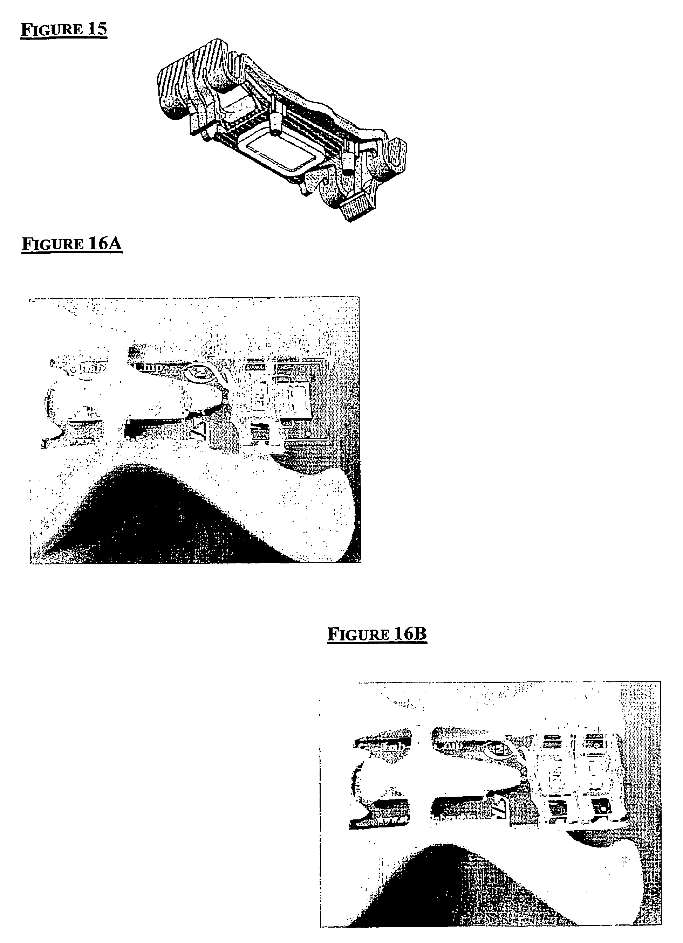

i) Prepare a new set of inlet (IN) and hybridization (clear) clamps.
ii) The hybridization clamp has a flat PDMS surface, 100 µm deep elicited in the gasket, and a surrounding trench used to accommodate the air displaced by the solution (Figure 15).
iii) Seal the inlets first using the IN clamp (Figure 16A).
iv) Seal the microarray chamber carefully using the hybridization clamp, making sure that no bubbles are introduced into the chamber (Figure 16B).
v) Tap the chip gently on the workbench, microarray closest to the bench surface, should any bubbles form during the sealing of the microarray chamber. This will force the bubbles to migrate to the outlet edge of the RT-PCR chamber where there are no probes.
vi) Remove the VereFlu^{™} Lab-On-Chip from its holder, making sure the clamps are tightly held in place.
vii) Load the sealed chip into the respective TCM. Press 'start' when prompted.
viii) When the hybridization is completed, remove the chip from TCM.

### Microarray Washing

Important Note: If case of precipitation or crystallization in the Wash Buffer Concentrate, warm up the entire bottle of buffer in a water bath set to 40°C for approximately 15 minutes or until the crystals dissolve. Mix thoroughly before use.

Measure 50 ml of the supplied Wash Buffer Concentrate into the provided 1 litre empty bottle. Top up the 1 litre bottle with DNase-/ RNase-free water to the 1000-ml mark.

Prepare and fill the supplied non-skirted 50 ml centrifuge tubes with 50 ml of the prepared Wash Buffer.

Insert the chip with the microarray end at the top into the centrifuge tube. Screw the tube cap on.

Place the tube with the microarray side facing towards the rotor into the centrifuge and centrifuge the tube at 3000 rpm for 1 min.

After the spin-dry, remove the chip and place it into the optical reader to analyze.

### Example 18. Analysis and Interpretation of Microarray Results

For any particular influenza type and strain, its respective probes will light up in a particular pattern on the microarray. A representative microarray picture using H5N 1 is shown in Figure 8.

For a sample to be positive for that particular influenza type, certain criteria must be satisfied. This criterion is called Diagnostic Rules and can be modified to suit different requirements. The VereID Platform Microarray Analysis software relies on this set of rules to determine the diagnosis of the sample.

The set of Diagnostic Rules for VereFlu^{™} can be found in the example below.

### Procedure:

Run the E@syCheck tool. A "New Report window" to create a new chip report is shown.
ii) Position the cursor on the text area in the "Lot" column. Use a barcode reader to scan the barcode placed on the LOC. The barcode can also be manually keyed into the text area.
ii) Insert the chip on the Optical Reader and perform an "Acquisition/Analysis".
   Note: Before the start of this operation, you can visualize the chip information obtained by the idp file:
iii) Choose the "Acquisition/Analysis" item by the ComboBox showed on the same row or pressing on the "Acquisition and Analysis" button on the toolbar or selecting the menu "Tools→ Acquisition and Analysis" item.

When "Acquisition/Analysis" operation starts, the progress bar near the ComboBox advances.

During this process, the following operations are performed:
If no error occurs during the image acquisition of lab-on-chip, the obtained image is automatically saved on disk in the folder "VereIDPlatform\Common\Sample" with the name "Lot-wafer-chip.tif'.

The image is analyzed using the image analysis protocol and the device files associated to the chip by the idp file or selected from the user.

### Example 19. Detection of Influenza Virus using VereChip System

In one embodiment, we describe a test system which may be performed in a single silicon chip integrating an ultra-fast miniaturized reactor for Reverse Transcription of RNA, PCR-based DNA amplification and a high-grade quality customised microarray for fast and accurate detection of Influenza. The system is referred to for convenience as the "VereFlu" system and should not be regarded as limiting the disclosure in this document.

We therefore disclose a method and kit for determining the presence or absence; and/or detection, differentiation and identification of an Influenza strain and/or subtype in a biological sample or from biological material isolated and/or purified from a biological sample.

We provide a novel approach by integrating PCR amplification and Microarray hybridization in a single silicon chip (VereFlu). This test combines the high sensitivity and specificity of the polymerase chain reaction (PCR) with the powerful identification capability of the microarray to provide answers to multiple questions within the shortest possible timeframe in a single test. By use of the probes and/or primers described herein, the method(s) and/or kit(s) are made more sensitive and/or specific than other detection methods of the prior art.

Acting as a fully integrated PCR reactor, the chip allows users to run multiplex amplification protocols for two genes (HA, NA) of influenza. Highly conductive material, integrated heaters and temperature sensors allow outstanding control and speed of the PCR temperature cycles, resulting in more specific PCR products in 45 to 100 minutes.

The diagnostic capability of the VereFlu Chip is an integration of the following 3 technologies:
1. Sample Preparation - by a RNA sample prep chip that will either be integrated into the entire device or a stand alone device. Currently, sample preparation is done off chip using commercial RNA extraction kits.
2. Multiplex RT-PCR - this is performed on the ultra-fast miniaturized reactor of the VereFlu Chip. PCR primers are designed to target the different genes of Influenza A (including its different subtypes) and Influenza B. Control primers and plasmid are also included as positive controls.
3.Microarray: DNA probes have been designed to detect the targeted genes of Influenza A (including its different subtypes) and Influenza B. The probes are spotted on the microarray chamber of the chip, where hybridization will occur with the amplified products from the PCR micro-reactor. The microarray results will be read by an optical reader which will enable the quick and accurate detection and identification of the Influenza subtype.

VereFlu may be used together with VereID^{™} platform that consists of: VereChip, VereID^{™} thermal cycler (TCS), VereID^{™} optical reader (OR) and VereID^{™} Biosystem software.

### Detection

After hybridization, the chip undergoes a brief wash prior to being analyzed in the Optical Reader.

A software system controls the operation of the entire test system. The software system analyses the image results obtained from the microarray.

The Easy-Check software automatically determines the specimen results. The interpretation of the assay specimen results is as follows:
An automatic grid alignment algorithm aligns a virtual grid over the microarray by using the corner features as reference.

Every cell of the virtual grid containing a feature is segmented by using the computation of the histogram and two percentile thresholds for identifying the background area and two percentile thresholds for identifying the signal area. The Microarray Layout is mapped onto the Virtual Grid. Each microarray feature name is connected with a specific cell of the virtual grid.

The signal area is used for computing the SIGNAL MEDIAN and other spot features, while the background area is used for computing the BACKGROUND MEDIAN and other spot features

The ON/OFF Rules are applied to assign to every spot the value of TURNED ON (symbol=1) or TURNED OFF (symbol=0)

### E.g. On/Off Rule: "Signal median /Background median> 3 * max (Signal med / Background med) of Negative Controls".

The matrix 1/0 (turned on/off) is submitted to a set of rules on controls.

E.g. Rule on control: "*Hybridization control turned on* = *11*" means that exactly 11 hybridization controls have to be turned on. All hybridization controls have counted without considering eventual replicas.

The matrix 1/0 (turned on/off) is submitted to a diagnostic rule. The diagnostic rule specifies, for each target, the minimum number of probes that must be turned on in order to give a positive result.

E.g. Diagnostic rule: "*Target H3N2: probe turned on* ≥ 4" means that the result will be positive if the SW find at list 4 probes related to H3N2 that are turned on.

### Example 20. Methods for Modifying or Adapting New Biomakers - Updating of the Flu Panel

To fully realize the diagnostic potential of the LOC platform, efforts will constantly be made to verify the relevance of the probe to current situations.

Established databases, e.g. NCBI, will be trawled for the latest updated sequences. Collaborations with WHO Influenza centers and government ministries will also enable up-to-date and crucial data to be made quickly available. Once any significant mutation occurs and is detected, new probes can be quickly designed and evaluated, following the probe design process, to detect the new emergent strain. The time taken for the discovery of a significant mutation to the updating of the microarray probe takes place in a short period of about a month. This process will allow the microarray panel to be as updated and relevant as possible in relation to the current ground situation.

Various modifications and variations of the described methods and system of the invention may be apparent to those skilled in the art without departing from the scope of the invention as defined in the appended claims. Although the invention has been described in connection with specific preferred embodiments, it should be understood that the invention as claimed should not be unduly limited to such specific embodiments.

## Claims

1. A combination of nucleic acids comprising primers of SEQ ID NO: 1 to SEQ ID NO: 16 as shown in Table D1, or a variant, homologue, derivative or fragment of at least 20 nucleotides in length thereof having at least 90% sequence identity thereto, for nucleic acid amplification of an influenza virus sequence, and probes of SEQ ID NO: 17 to SEQ ID NO: 53 as shown in Table D2, or a variant, homologue, derivative or fragment of at least 20 nucleotides in length thereof having at least 90% sequence identity thereto, wherein the probes are immobilised and are capable of hybridising to an influenza virus sequence.

2. The combination of claim 1, wherein the probes are immobilised in the form of an array.

3. The combination of claim 2, wherein the array is a microarray.

4. A combination according to any preceding claim, in which the combination further comprises one or more of the primer sequences set out in Table D3.

5. A combination according to any preceding claim, in which the combination further comprises one or more of the probe sequences set out in Table D4.

6. The combination of any previous claim for use on a device comprising a first chamber for nucleic acid amplification of the influenza virus sequence using the primers and a second chamber for nucleic acid hybridisation of the influenza virus sequence using the immobilised probes.

7. The combination of claim 6, wherein the first and second chambers of the device are in fluid connection.

8. A method of detecting an influenza virus sequence in a biological sample and/or determining the strain of an influenza virus sequence in a biological sample, comprising:
a) performing a nucleic acid amplification comprising amplifying the influenza virus sequence to produce amplicons using primers of SEQ ID NO: 1 to SEQ ID NO: 16 as shown in Table D1, or a variant, homologue, derivative or fragment of at least 20 nucleotides in length thereof having at least 90% sequence identity thereto;
b) performing a nucleic acid hybridisation comprising hybridising the amplified influenza virus sequence to immobilised probes of SEQ ID NO: 17 to SEQ ID NO: 53 as shown in Table D2, or a variant, homologue, derivative or fragment of at least 20 nucleotides in length thereof having at least 90% sequence identity thereto; and
c) detecting binding between the immobilised probe and the amplified influenza virus sequence.

9. The method of claim 8, wherein the nucleic acid amplification step a) is performed in a first chamber of a device, and the nucleic acid hybridisation step b) is performed in a second chamber of the device.

10. The method of claim 8 or claim 9, wherein the method is capable of multiplex detection of targets.

11. The method of any of claims 8 to 10, wherein the nucleic acid amplification step a) further comprises amplifying the influenza sequence using one or more of the primer sequences set out in Table D3.

12. The method of any of claims 8 to 11, wherein the nucleic acid hybridisation step b) further comprises hybridising the influenza sequence using one or more of the probe sequences set out in Table D4.

## Patentansprüche

1. Kombination von Nukleinsäuren umfassend Primer mit SEQ ID No: 1 bis SEQ ID No: 16, wie in Tabelle D1 gezeigt, oder einer Variante, einem Homolog, einem Derivat oder einem Fragment davon mit einer Länge von wenigstens 20 Nukleotiden mit wenigstens 90 % Sequenzidentität dazu, für Nukleinsäureamplifikation einer Influenzavirussequenz, und Sonden mit SEQ ID No: 17 bis SEQ ID No: 53, wie in Tabelle D2 gezeigt, oder einer Variante, einem Homolog, einem Derivat oder einem Fragment davon mit einer Länge von wenigstens 20 Nukleotiden mit wenigstens 90 % Sequenzidentität dazu, wobei die Sonden immobilisiert sind und in der Lage sind, mit einer Influenzavirussequenz zu hybridisieren.

2. Kombination nach Anspruch 1, wobei die Sonden in der Form eines Arrays immobilisiert sind.

3. Kombination nach Anspruch 2, wobei der Array ein Mikroarray ist.

4. Kombination nach einem jeden vorhergehenden Anspruch, wobei die Kombination weiter eine oder mehrere der Primersequenzen umfasst, die in Tabelle D3 angegeben sind.

5. Kombination nach einem jeden vorhergehenden Anspruch, wobei die Kombination weiter eine oder mehrere der Sondensequenzen umfasst, die in Tabelle D4 angegeben sind.

6. Kombination nach einem jeden vorhergehenden Anspruch zur Verwendung in einer Vorrichtung umfassend eine erste Kammer für Nukleinsäureampliflikation der Influenzavirussequenz unter Verwendung der Primer und eine zweite Kammer für Nukleinsäurehybridisierung der Influenzavirussequenz unter Verwendung der immobilisierten Sonden.

7. Kombination nach Anspruch 6, wobei die erste und zweite Kammer der Vorrichtung in Flüssigverbindung stehen.

8. Verfahren zum Nachweisen einer Influenzavirussequenz in einer biologischen Probe und/oder Bestimmen des Stammes einer Influenzavirussequenz in einer biologischen Probe, umfassend:
a) Durchführen einer Nukleinsäureamplifikation umfassend Amplifizieren der Influenzavirussequenz, um Amplicons zu erzeugen, unter Verwendung von Primern mit SEQ ID No: 1 bis SEQ ID No: 16, wie in Tabelle D1 gezeigt, oder einer Variante, einem Homolog, einem Derivat oder einem Fragment davon mit einer Länge von wenigstens 20 Nukleotiden mit wenigstens 90 % Sequenzidentität dazu;
b) Durchführen einer Nukleinsäurehybridisierung umfassend Hybridisieren der amplifizierten Influenzavirussequenz an immobilisierten Sonden mit SEQ ID No: 17 bis SeEQ ID No: 53, wie in Tabelle D2 gezeigt, oder einer Variante, einem Homolog, einem Derivat oder einem Fragment davon mit einer Länge von wenigstens 20 Nukleotiden mit wenigstens 90 % Sequenzidentität dazu; und
c) Nachweisen von Binden zwischen der immobilisierten Sonde und der amplifizierten Influenzavirussequenz.

9. Verfahren nach Anspruch 8, wobei der Nukleinsäureamplifikationsschritt a) in einer ersten Kammer der Vorrichtung durchgeführt wird und der Nukleinsäurehybridisierungsschritt b) in einer zweiten Kammer der Vorrichtung durchgeführt wird.

10. Verfahren nach Anspruch 8 oder Anspruch 9, wobei das Verfahren zu einem Multiplex-Nachweis von Zielmolekülen in der Lage ist.

11. Verfahren nach einem der Ansprüche 8 bis 10, wobei der Nukleinsäureamplifikationsschritt a) weiter Ampflizieren der Influenzasequenz umfasst unter Verwendung von einer oder mehreren der in Tabelle D3 angegebenen Primersequenzen.

12. Verfahren nach einem der Ansprüche 8 bis 11, wobei der Nukleinsäurehybridisierungsschritt b) weiter Hybridisieren der Influenzasequenz umfasst unter Verwendung von einer oder mehreren der in Tabelle D4 angegebenen Sondensequenzen.

## Revendications

1. Combinaison d'acides nucléiques comprenant les amorces de SEQ ID NO: 1 à SEQ ID NO: 16 telles que montrées dans le Tableau D1, ou un variant, homologue, dérivé ou fragment de celles-ci d'une longueur d'au moins 20 nucléotides présentant au moins 90 % d'identité de séquence avec celles-ci, pour l'amplification d'acide nucléique d'une séquence du virus de la grippe, et les sondes de SEQ ID NO: 17 à SEQ ID NO: 53 telles que montrées dans le Tableau D2, ou un variant, homologue, dérivé ou fragment de celles-ci d'une longueur d'au moins 20 nucléotides présentant au moins 90 % d'identité de séquence avec celles-ci, où les sondes sont immobilisées et sont capables de s'hybrider à une séquence du virus de la grippe.

2. Combinaison selon la revendication 1, dans laquelle les sondes sont immobilisées sous la forme d'une série.

3. Combinaison selon la revendication 2, dans laquelle la série est une bioprice.

4. Combinaison selon l'une quelconque des revendications précédentes, où la combinaison comprend en outre une ou plusieurs des séquences d'amorces décrites dans le Tableau D3.

5. Combinaison selon l'une quelconque des revendications précédentes, où la combinaison comprend en outre une ou plusieurs des séquences de sondes décrites dans le Tableau D4.

6. Combinaison selon l'une quelconque des revendications précédentes, destinée à être utilisée sur un dispositif comprenant une première chambre pour l'amplification d'acide nucléique de la séquence du virus de la grippe en utilisant les amorces, et une seconde chambre pour l'hybridation d'acide nucléique de la séquence du virus de la grippe en utilisant les sondes immobilisées.

7. Combinaison selon la revendication 6, dans laquelle la première et la seconde chambre du dispositif sont en connexion fluidique.

8. Procédé pour détecter une séquence du virus de la grippe dans un échantillon biologique et/ou déterminer la souche d'une séquence du virus de la grippe dans un échantillon biologique, qui consiste à :
a) réaliser une amplification d'acide nucléique qui consiste à amplifier la séquence du virus de la grippe afin de produire des amplicons en utilisant les amorces de SEQ ID NO: 1 à SEQ ID NO: 16 telles que montrées dans le Tableau D1, ou un variant, homologue, dérivé ou fragment de celles-ci d'une longueur d'au moins 20 nucléotides présentant au moins 90 % d'identité de séquence avec celles-ci ;
b) réaliser une hybridation d'acide nucléique qui consiste à hybrider la séquence du virus de la grippe amplifiée à des sondes immobilisées de SEQ ID NO: 17 à SEQ ID NO: 53 telles que montrées dans le Tableau D2, ou un variant, homologue, dérivé ou fragment de celles-ci d'une longueur d'au moins 20 nucléotides présentant au moins 90 % d'identité de séquence avec celles-ci ; et
c) détecter une liaison entre la sonde immobilisée et la séquence du virus de la grippe amplifiée.

9. Procédé selon la revendication 8, dans lequel l'étape a) d'amplification d'acide nucléique est réalisée dans une première chambre d'un dispositif, et l'étape b) d'hybridation d'acide nucléique est réalisée dans une seconde chambre du dispositif.

10. Procédé selon la revendication 8 ou la revendication 9, où le procédé est capable de réaliser une détection de cibles multiplexe.

11. Procédé selon l'une quelconque des revendications 8 à 10, dans lequel l'étape a) d'amplification d'acide nucléique consiste en outre à amplifier la séquence de la grippe en utilisant une ou plusieurs des séquences d'amorces décrites dans le Tableau D3.

12. Procédé selon l'une quelconque des revendications 8 à 11, dans lequel l'étape b) d'hybridation d'acide nucléique consiste en outre à hybrider la séquence de la grippe en utilisant une ou plusieurs des séquences de sondes décrites dans le Tableau D4.
